# EUROPEAN PATENT APPLICATION

(11) **EP 2 700 933 A1**
(43) Date of publication of application: **26.02.2014**
(21) Application number: 12382330.4
(22) Date of filing: 20.08.2012
(51) Int. Cl.: G01N 21/35, G01N 21/65

(54) **Raman, infrared, or Raman-Infrared analysis of peripheral blood plasma protein structure and its relation to cognitive development in Alzheimer's disease**

(71) Applicant: Consejo Superior De Investigaciones Científicas (CSIC), 28006 Madrid (ES); Fundación Investigación Biomédica Hospital Universitario 12 Octubre, 28041 Madrid (ES); Instituto de Salud Carlos III, 28029 Madrid (ES); Fundación Cien, 28031 Madrid (ES); Universidad Complutense De Madrid, 28040 Madrid (ES)
(72) Inventor: Carmona Hernández, Pedro, 28006 Madrid (ES); Toledano Gasca, Adolfo, 28006 Madrid (ES); Calero Lara, Miguel, 28029 Madrid (ES); Martínez Martín, Pablo, 28029 Madrid (ES); Bermejo Pareja, Félix, 28041 Madrid (ES); Molina Santos, Marina, 28040 Madrid (ES)
(74) Representative: ABG Patentes, S.L.

(57) **Abstract**

The present invention relates to a Raman spectroscopy method for determining protein structure associated with global cognitive deterioration in Alzheimer's disease from the Raman spectroscopic analysis of a human blood sample, preferably of a human blood plasma sample, comprising obtaining at least one spectral value of at least one of the Raman spectrum regions comprised between 1600-1700 cm⁻¹, between 910-980 cm⁻¹, between 730-760 cm⁻¹ and/or between 390-450 cm⁻¹, and where said spectral value allows obtaining an indication of the presence or absence of protein structure associated with a condition of Alzheimer's disease. The present invention also relates to a method for the infrared spectroscopic analysis of a blood sample, by means of additionally obtaining at least one spectral value of at least one of the infrared spectrum regions comprised between 1600 and 1700 cm⁻¹ and/or between 1000 and 1150 cm⁻¹. Optionally, the Raman spectroscopy method further comprises the infrared spectroscopic analysis of the same blood sample. The invention also relates to a diagnostic method for diagnosing Alzheimer's disease comprising measuring a Raman spectrum and/or an infrared spectrum of a plasma sample.

## Description

### Field of the Invention

The present invention relates to a new method for the analysis of plasma proteins by vibrational spectroscopy (FT-Raman, FT-Infrared or FT-Raman in combination with FT-Infrared), whereby demonstrating the existence of a correlation between secondary and tertiary protein structure and cognitive development in Alzheimer's disease (AD), the main sector of application being the health sector. The invention particularly relates to a diagnostic method for diagnosing AD based on the vibrational spectroscopy, and it may also be of interest for the commercial sector relating to this spectroscopic instrumentation.

### Background of the Invention

It is envisaged that the number of people with dementia, 25 million worldwide in the year 2000, will increase up to 63 million in 2030 and 114 million in 2050 as a result of demographic changes and increased longevity. There is a wide range of complex neurodegenerative disorders, all of which are characterized by neuronal dysfunctions finally leading to neuronal death, associated with the development of these diseases. Alzheimer's disease (AD) is the most common. It has been calculated that over half the patients with dementia have AD, and this number will double every twenty years. Though the clinical signs of neurodegenerative dementias are often related to the most affected area of the brain, the onset of mixed pathologies is common and the clinical manifestations in early stages are often similar. Furthermore, a single type of pathology may produce different cognitive results, making diagnosis difficult. There is evidence that pathological changes in diseases presenting dementia start decades before the onset of the first clinical manifestations. The challenge of finding effective treatments for AD and other dementias is parallel to exploring the detection of preclinical changes as early as possible and accurately identifying the pathology (or pathologies) responsible.

Accordingly, these considerations pose enormous challenges to society and health systems, and hence the need for research in biomarkers that allow the early detection of these diseases in order to administer a suitable treatment and accurately track their progression. The most effective definitive diagnosis of AD today is limited to an autopsy, and the clinical diagnosis is done by exclusion after a specialist analyzes whether dementia is present and finds no symptom or sign explaining the cause of the dementia. There are three fields where results have theoretically or practically been obtained that are useful for developing diagnostic tests: genetics, biomarker detection/quantification, neuroimaging. All these lines, some of which are very expensive, such as neuroimaging, have yielded certain success but have still not provided diagnostic protocols to surpass 80% diagnostic reliability and specificity needed in medical practice. There are many problems posed by AD, including early diagnosis and tracking the progression of patients, as well as definitive post-mortem diagnosis and neuropathological change characterization. All this requires developing sensitive, reliable, easy to perform and repeat and low-cost methods.

Even though the causes of AD are unknown, and there is discussion as to the "cascade/ cascades of pathogenic event/events" leading to the final neurodegenerative stage in AD, there is sufficient knowledge about the involutional processes that occur in order to be able to define possible biomarkers indicative of the disease. It is well-known that one of the most important pathological characteristics of AD is the accumulation of fibrillar proteins in the brain which is expressed as the formation of (extraneuronal) senile plaques and (intraneuronal) neurofibrillary tangles the primary component of which is Aβ-amyloid peptide. Some authors have proven concentrations of some amyloid peptides in peripheral blood, which is an easy to extract biological fluid, to be correlated with certain stages of the disease or with the risk of suffering it, having a predictive value in certain cases (Graff-Radford et al. Arch. Neurol. 64, 354-62 (2007); Baranowska-Bik et al., Neuro. Endocrinol. Lett. 29, 75-79 (2008); Modrego et al., Am. J. Alzheimers Dis. Other Demen. 23, 286-290 (2008)). In other studies, certain biomarkers relating to oxidative stress, either free radicals or peroxidized macromolecules (lipids, proteins, DNA of blood cells) or systems for producing or neutralizing radicals (superoxide dismutase enzymes, etc.), have been found to increase in the blood of Alzheimer's patients (Baldeiras et al. Alzheimers Dis. 15, 117-128 (2008); Bermejo et al. Free Rad. Res. 42, 162-170 (2008); Greibelger et al. Free Rad. Res. 42, 633-638 (2008)).

Among the numerous research laboratory diagnostic techniques, vibrational spectroscopy has the advantage of being rapid, non-invasive and relatively low-cost. It also provides analytical and structural information about the biological system molecular components, which makes it useful in these applications. Raman spectroscopy has been applied for AD diagnosis by means of measuring spectra in brain tissue, Aβ-amyloid peptide deposit, increase in cholesterol and hyperphosphorylated tau protein being found as biochemical changes (Archer et al. European Conference on Biomedical Optics (ECBO), Munich, Germany, 2007; Chen et al. Appl. Optics 48, 4743-4748 (2009)). However, this work omits specificity and sensitivity of this technique in detecting AD in brain tissue, and furthermore this tissue is not as accessible as peripheral blood, which is known to be an easy to extract biological fluid. Furthermore, changes occurring in brain tissue do not necessarily have to occur in peripheral blood as well.

Raman spectroscopy has also been used to study peripheral blood platelets for AD diagnosis purposes (Chen et al. Laser Phys. Lett. 8, 547-552 (2011)). This paper does, however, present the following drawbacks:
- The use of a 633 nm excitation laser line, like other visible spectrum laser lines, can generate fluorescence in biological substances which at least partially masks the Raman spectra of the samples in question, with their subsequent analytical uselessness. Due to a Raman resonance effect, Raman excitation with visible spectrum laser lines can cause an enormous intensification of the bands of the colored minority plasma components (carotenoids, bilirubin, hemoglobin) which can greatly mask the majority components of this fraction (proteins, lipids). And if the content of these colored minority components is erratic, the subsequent intense Raman signals will also be erratic, which will therefore complicate correct sample classification. Hence the need to use more practical lasers for diagnostic purposes, with excitation in the near-infrared associated with Fourier transform-Raman spectrometers (FT-Raman spectrometers) minimizing fluorescence probability of most samples Applications of Vibrational Spectroscopy in Pharmaceutical Research and Development". Chichester, West Sussex, England, 2007, 353-362.
- The platelet samples analyzed by Raman spectroscopy in that paper were from mice transgenic, the proteins of which are not identical to human proteins. Therefore the analytical-structural changes in the course of AD of these rodents can be different from those occurring in human peripheral blood, as has been demonstrated in the present invention. Furthermore, the sample size in the mentioned spectroscopic platelet research was very small (10 mice with AD and 8 healthy controls), and none of the current mice transgenic models with AD is perfectly comparable to humans with this disease (Wisniewski and Sigurdssson, Biochim. Biophys. Acta 1802, 847-859 (2010)).

In turn, and for the purpose of diagnosing AD, transmission infrared (IR) spectroscopy of mononuclear leukocytes has also been used (Carmona et al. Anal. Bioanal. Chem. 402, 2015-2021 (2012)). By using β-sheet structure percentage as a single diagnostic parameter, the resulting sensitivity is close to 80% in classifying samples belonging to groups of healthy controls and to patients with mild, moderate and severe AD. However, infrared spectroscopy alone in this sense has a drawback with respect to Raman spectroscopy that can be described as follows. Even though water is found in minor amounts in dry samples at room temperature, it causes background absorption below 850 cm⁻¹ masking the bands of the biological substances of the sample in question. This means that the spectral range available for sample analysis is shorter in the infrared than in Raman, and therefore provides less information. Furthermore, among the macromolecular structure-inherent factors and spectroscopic selection rules it must be pointed out that infrared is a technique providing information about secondary protein structure, but not about the tertiary structure of side chains containing tryptophan, phenylalanine, tyrosine, cystine/cysteine.

Therefore, there is a need to develop analytical techniques that allow finding blood protein structure and oxidative stress patterns linked to cognitive deterioration associated with Alzheimer's disease which allow more precisely distinguishing a condition of said disease.

### Brief Description of the Invention

The present invention relates to the Raman, infrared or Raman/infrared analysis of peripheral blood plasma samples and correlation with cognitive development in Alzheimer's disease. As shown in Examples 1 and 2, prior blood sample fractionation is possible for carrying out the methods of the invention, these fractionated samples being collected in heparinized tubes and subjected to centrifugation. Approximately 50 microliters of the supernatant are deposited on a sample holder and dried at room temperature for approximately 30 minutes. The Raman spectrum of the resulting solid sample is measured after homogenization. The spectrum is normalized and the baseline is corrected to subsequently determine changes in spectroscopic parameters (frequencies, ratio of band heights and areas) caused by the formation of Aβ-amyloid peptides and/or by changes in the secondary and tertiary protein structure in the course of AD. The spectroscopic parameters are subsequently subjected to discriminant analysis, which is based on correlating certain spectral regions with the presence or absence of AD. These regions in Raman spectroscopy are the amide I region (1700-1600 cm⁻¹), the region comprised between 980 and 910 cm⁻¹ (including typical C-C stretching bands of α-helices), the 760-730 cm⁻¹ region of tertiary protein structure with tryptophan residues, the 450-390 cm⁻¹ region of angular deformation, and the C-H stretching region comprised between 3100 and 2800 cm⁻¹. The suitable regions in infrared spectroscopy are the amide I region (1700-1600 cm⁻¹) for protein structure and the region comprised between 1150 and 1000 cm⁻¹ for oxidative stress. The discriminant analysis of spectroscopic parameters of these spectral regions allows classifying the plasma samples from healthy subjects or from AD patients with sensitivity and specificity greater than 90%.

In one aspect, the invention relates to a vibrational spectroscopy method for determining protein structure associated with global cognitive deterioration in Alzheimer's disease in a blood sample comprising the following steps:
a) recording a Raman spectrum of a previously obtained human blood sample;
b) obtaining a Raman spectral value of at least one of the following Raman spectrum regions:
   R.1. region comprised between 1600 and 1700 cm⁻¹;
   R.2. region comprised between 910 and 980 cm⁻¹;
   R.3. region comprised between 730 and 760 cm⁻¹;
   R.4. region comprised between 390 and 450 cm⁻¹;
      where said spectral value is selected from a value of an interband Raman intensity ratio, a value of the area of said spectral region and/or a frequency value.
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
   by comparison of the Raman spectral value obtained in step b with a reference spectral value which allows distinguishing between class I or II, or by multivariate analysis comparison of the Raman spectral value obtained in step b with class I and class II reference Raman spectral values.

In another aspect, the invention relates to a vibrational spectroscopy method for determining protein structure associated with global cognitive deterioration in Alzheimer's disease in a blood sample comprising the following steps:
a) recording an infrared spectrum of a previously obtained human blood sample;
b) obtaining an infrared spectral value of at least one of the following infrared spectrum regions:
   IR.1. region comprised between 1600 and 1700 cm⁻¹;
   IR.2. region comprised between 1000 and 1150 cm⁻¹;
      where said infrared spectral value is selected from a value of the area of said spectral region and/or a percentage value of the interband areas of said spectral region;
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
by comparison of the infrared spectral value obtained in step b with a reference infrared value which allows distinguishing between class I or II, or by multivariate analysis comparison of the infrared value obtained in step b with class I or II reference infrared spectral values.

In another aspect, the invention relates to a diagnostic method for diagnosing Alzheimer's disease in a subject comprising the steps of:
a) measuring a Raman spectrum of a plasma sample from said subject obtaining at least one Raman band selected from the group consisting of a Raman band comprising vibrations specific to β-protein structure, a Raman band of amyloid peptides comprising vibrations specific to angular deformation of the peptide bond, a Raman band comprising vibrations specific to α-helix protein structure and a Raman band comprising vibrations specific to tertiary protein structure with tryptophan residues and
b) comparing the Raman spectrum obtained in step a) with the spectrum of a reference sample
wherein a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum and/or a Raman spectrum variation generated by vibrations specific to tryptophan residues in a tertiary protein structure with respect to the reference spectrum is indicative of the patient having Alzheimer's disease.

In another aspect, the invention relates to a diagnostic method for diagnosing Alzheimer's disease in a subject comprising the steps of:
a) measuring an IR spectrum of a plasma sample from said subject obtaining at least one band specific to the presence of β-protein structure and/or in at least one specific band indicative of the presence of compounds generated during oxidative stress and
b) comparing the IR spectrum obtained in step (a) with the spectrum of a reference sample
wherein an IR spectrum variation indicative of an increase in β-protein structure with respect to the reference spectrum and/or an IR spectrum variation indicative of an increase in the concentration of compounds generated in the sample during oxidative stress with respect to the reference spectrum is indicative of the patient having Alzheimer's disease.

### Detailed Description of the Drawings

Figure 1 shows Raman spectra, in the 3600-300 cm⁻¹ region, of peripheral blood plasma obtained from a healthy control (a) and from patients with mild (b), moderate (c) and severe (d) AD.
Figure 2 shows Raman spectra, in the 1720-1600 cm⁻¹ region, of peripheral blood plasma obtained from a healthy control (-) and from patients with mild (- - -) and moderate (......) AD.
Figure 3 shows Raman spectra, in the 450-380 cm⁻¹ region, of peripheral blood plasma obtained from a healthy control (-) and from patients with mild (- - -) and moderate (------) AD.
Figure 4 shows Raman spectra, in the 1000-900 cm⁻¹ region, of peripheral blood plasma obtained from a healthy control (-) and from patients with mild (- - -), moderate (......) and severe (-.-.-.) and.
Figure 5 shows Raman spectra, in the 780-720 cm⁻¹ region, of peripheral blood plasma obtained from a healthy control (-) and from patients with mild (- - -), moderate (......) and severe (-.-.-.) and.
Figure 6 shows infrared spectra of plasma from senile controls (-) and plasma from patients with moderate (- - - - -) AD. (A): amide I region. Original spectra (upper) and spectra expressed in second derivatives (lower). (B): region between 1150-1000 cm⁻¹.

### Detailed Description of the Invention

Methods for determining protein structure associated with global cognitive deterioration in Alzheimer's disease and for diagnosing Alzheimer's disease

The problem in obtaining a method for determining the global deterioration of AD and for diagnosing AD has been solved according to the method of the invention by means of the analytical application of Raman spectroscopy which provides spectral parameters relating to secondary and tertiary protein structure which, in combination with infrared spectroscopy techniques, further provides additional information relating to oxidative stress, as shown in Examples 3 to 6 of the invention.

The basis of the invention consists of the β-sheet protein structure and oxidative stress being more abundant in plasma obtained from patients with the AD than in that obtained from healthy controls, the content of α-helix structure being lower in patients with AD with respect to healthy controls and, on the other hand, of there being a certain correlation between the Global Deterioration Scale (GDS) of this disease and the infrared spectral profiles between 1700-1600 cm⁻¹ (amide I) and between 1150-1000 cm⁻¹, and the Raman spectral profiles of the amide I (1700-1600 cm⁻¹) region, the region comprised between 980 and 910 cm⁻¹ (including characteristic C-C stretching bands of α-helices), the 760-730 cm⁻¹ region of tertiary protein structure with tryptophan residues, the 450-390 cm⁻¹ region of angular deformation, and the C-H stretching region comprised between 3100 and 2800 cm⁻¹.

As previously mentioned, Raman spectroscopy provides analytical-structural information about the secondary protein structure and about the tertiary structure of side chains containing tryptophan, phenylalanine, tyrosine, cystine/cysteine. One of the factors contributing to the distinction between plasma samples from healthy subjects and Alzheimer's patients is demonstrated in the present invention by Raman spectroscopy to be not only the amide I band, but also bands due to the tertiary structure of side chains of tryptophan and others located below 500 cm⁻¹ that are not visible by infrared spectroscopy. In fact, one of the advantages of the present invention consists of a new parameter or spectroscopic marker in the 500-400 cm⁻¹ region, attributable to Aβ-amyloid peptides, for sample classification having been detected for the first time. On the other hand, infrared spectroscopy also provides information about protein structure in the amide I region and about the oxidative stress associated with AD in the region between 1150-1000 cm⁻¹.

In the scope of the present invention, the global cognitive deterioration scale (also referred to herein as GDS) relates to the Global Deterioration Scale defined by Reisberg to describe in detail the stages of Alzheimer's disease progression. Said scale consists of seven levels, and in the scope of the present invention they are defined as:
- GDS-1: corresponding to a normal individual without cognitive decline and a normal clinical phase;
- GDS-2: corresponding to an individual with a level of very mild cognitive decline (subjective cognitive deterioration) and a clinical phase of forgetfulness;
- GDS-3: corresponding to an individual with a level of mild cognitive decline and a clinical phase of early confusion;
- GDS-4: corresponding to an individual with a level of moderate cognitive decline and a clinical phase of late confusion;
- GDS-5: corresponding to an individual with a level of moderately severe cognitive decline and a clinical phase of early dementia;
- GDS-6: corresponding to an individual with a level of severe cognitive decline and a clinical phase of mild dementia;
- GDS-7: corresponding to an individual with a level of very severe cognitive decline and a clinical phase of severe dementia.

For purposes of this specification, GDS-3 stage is considered mild Alzheimer's disease (mild AD) development level, the GDS-4 and GDS-5 stages are considered a moderate Alzheimer's disease (moderate AD) development level and the GDS-6 and GDS-7 stages are considered an advanced, serious or severe Alzheimer's disease (severe AD) development level.

A first aspect of the invention relates to a method (first method of the invention) for determining protein structure associated with global cognitive deterioration in Alzheimer's disease in a blood sample by means of vibrational spectroscopy, comprising the following steps:
a) recording a Raman spectrum of a previously obtained human blood sample;
b) obtaining a Raman spectral value of at least one of the following Raman spectrum regions:
   R.1. region comprised between 1600 and 1700 cm⁻¹;
   R.2. region comprised between 910 and 980 cm⁻¹;
   R.3. region comprised between 730 and 760 cm⁻¹;
   R.4. region comprised between 390 and 450 cm⁻¹;
      where said spectral value is selected from a value of an interband Raman intensity ratio, a value of the area of said spectral region and/or a frequency value.
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
by comparison of the Raman spectral value obtained in step b with a reference spectral value which allows distinguishing between class I or II, or by multivariate analysis comparison of the Raman spectral value obtained in step b with class I and class II reference Raman spectral values.

In the scope of the present invention, the expression "Raman spectrum" refers to a Raman spectrum comprising the spectral region comprised between 390 and 1700 cm⁻¹, being able to have a broader spectral region comprising said previous spectral region. In fact, the authors of the present invention have found the presence of 5 spectral regions in the spectral region comprised between 300 and 3600 cm⁻¹ which are correlated with the presence or absence of AD, comprising the regions defined in R.1 to R.4, as well as R.5 region corresponding to the C-H stretching region comprised between 3100 and 2800 cm⁻¹. In such case, to include said R.5 region in the analysis, it would be convenient for said Raman spectrum to comprise the spectral region between 390 and 3100 cm⁻¹. To obtain better results with the method that is described in this invention, it is convenient to remove fluorescence signals generated in the spectrum by biological substances contained in the blood samples which at least partially mask the Raman spectra of the samples in question. Though fluorescence of the Raman spectra generated with Raman excitation with laser lines in the visible spectrum can be minimized by means of optimizing the microspectrometer to be used, the fluorescence suppression cannot be 100%, it being convenient to obtain the spectrum by means of using laser lines with near-infrared excitation, such as a 1064 nm neodymium-YAG laser line for example. Furthermore, the use of near-IR lasers eliminates the harmful Raman resonance of the colored minority components which can also mask the protein and lipid spectra.

In a particular embodiment of the methods of the invention comprising recording a Raman spectrum, said Raman spectrum is recorded using a near-infrared excitation line.

In another particular embodiment, the first method of the invention comprises determining a combination of spectral values of two or more regions. In a particular embodiment, the first method of the invention comprises determining the spectral value of any one combination of two Raman spectrum regions selected from the group consisting of the region comprised between 1600 and 1700 cm⁻¹ and the region comprised between 910 and 980 cm⁻¹; the region comprised between 1600 and 1700 cm⁻¹ and the region comprised between 730 and 760 cm⁻¹; the region comprised between 1600 and 1700 cm⁻¹ and the region comprised between 390 and 450 cm⁻¹; the region comprised between 910 and 980 cm⁻¹ and the region comprised between 730 and 760 cm⁻¹; the region comprised between 910 and 980 cm⁻¹ and the region comprised between 390 and 450 cm⁻¹; the region comprised between 730 and 760 cm⁻¹ and the region comprised between 390 and 450 cm⁻¹. In another particular embodiment, the first method of the invention comprises determining a spectral value of any one combination of three Raman spectrum regions selected from the group consisting of the region comprised between 1600 and 1700 cm⁻¹, the region comprised between 910 and 980 cm⁻¹ and the region comprised between 730 and 760 cm⁻¹; the region comprised between 1600 and 1700 cm⁻¹, the region comprised between 910 and 980 cm⁻¹ and the region comprised between 390 and 450 cm⁻¹; the region comprised between 1600 and 1700 cm⁻¹, the region comprised between 730 and 760 cm⁻¹ and the region comprised between 390 and 450 cm⁻¹; the region comprised between 910 and 980 cm⁻¹, the region comprised between 730 and 760 cm⁻¹ and the region comprised between 390 and 450 cm⁻¹. In another particular embodiment, the first method of the invention comprises obtaining the Raman spectral value of four Raman spectrum regions, specifically the region comprised between 1600 and 1700 cm⁻¹, the region comprised between 910 and 980 cm⁻¹, the region comprised between 730 and 760 cm⁻¹ and the region comprised between 390 and 450 cm⁻¹.

According to the invention, the complementary IR spectroscopic study of the same blood sample, preferably of the same blood plasma sample, can provide additional information for determining the presence of protein structure associated with global cognitive deterioration in Alzheimer's disease by means of analyzing the amide I region of its IR spectrum. Furthermore, said IR spectrum also provides another additional marker in the region between 1150-1000 cm⁻¹, which has a certain correlation with the Global Deterioration Scale (GDS) of this disease and relates to the oxidative stress associated with AD, its use being convenient for classifying said samples according to their content of protein structure associated with Alzheimer's disease.

In another aspect, the invention relates to a vibrational spectroscopy method (second method of the invention) for determining protein structure associated with global cognitive deterioration in Alzheimer's disease in a blood sample, comprising the following steps:
a) recording an infrared spectrum of a previously obtained human blood sample;
b) obtaining an infrared spectral value of at least one of the following infrared spectrum regions:
   IR.1. region comprised between 1600 and 1700 cm⁻¹;
   IR.2. region comprised between 1000 and 1150 cm⁻¹;
   where said infrared spectral value is selected from a value of the area of said spectral region and/or a percentage value of the interband areas of said spectral region;
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
by comparison of the infrared spectral value obtained in step b with a reference infrared value which allows distinguishing between class I or II, or by multivariate analysis comparison of the infrared value obtained in step b with class I or II reference infrared spectral values.

In the scope of the present invention, the expression "infrared (IR) spectrum" refers to an IR spectrum comprising the spectral regions between 1000 and 1150 cm⁻¹ and between 1600-1700 cm⁻¹, said spectrum being able to consist of a broader spectral region comprising the previous spectral regions, those spectra comprising at least the spectral region between 910 and 1700 cm⁻¹ being preferred.

In another aspect, the invention relates to a method (third method of the invention), applied to a sample from the same subject, according to the first method of the invention additionally comprising the steps of the second method of the invention. In a particular embodiment of the third method of the invention, the sample is classified by means of the multivariate analysis comparison of the spectral values obtained in step b with class I and class II reference Raman values and with class I and class II reference infrared spectral values.

In the scope of the present invention, "multivariate analysis comparison" refers to comparison by means of one of these three alternative methods: discriminant analysis, cluster analysis and neural network analysis. Discriminant analysis comparison is preferred as a model made up of a discriminant function (for two groups in this invention) based on linear combinations of predictor variables providing the best discrimination possible between groups, because said discriminant analysis provides better sample classification results than those obtained by means of the other alternative methods for comparison.

A person skilled in the art will understand that according to the methods of the invention, after recording a Raman spectrum according to the methods of the invention or after recording an infrared spectrum according to the methods of the invention, in a preferred embodiment, the invention contemplates correcting the baseline of the spectrum and normalizing said spectrum with respect to the area of the amide I band.

As it is used in the present invention, "amide I band" refers to the peptide and protein band primarily consisting of the polypeptide backbone carbonyl group stretching vibrations. It is a sensitive marker of the secondary structure of the polypeptide backbone, because since the vibration frequency of each C=O bond depends on hydrogen bonds and on interactions between amide groups, both are influenced by the secondary structure.

As understood in the invention, the blood sample analyzed is a human blood sample already obtained (i.e., previously extracted from a subject), human peripheral blood samples being preferred because they are easier to obtain. Human blood is made up of an acellular fraction (blood plasma) and various cellular fractions (such as for example red blood cells, leukocytes) and platelet elements, which can be used in the scope of the present invention as the blood sample to be analyzed, a blood plasma fraction sample being preferred for carrying out the aforementioned method of analysis.

As it is understood in the invention, "plasma" refers to the liquid element of blood, representing 60% of the total blood volume and lacking red and white blood cells. The plasma fraction is preferred over the platelet fraction because it is easier to obtain, besides the fact that the difficulties in obtaining platelets due to platelet aggregation or rupture phenomena are well-known.

The present invention can be carried out on substantially platelet-free plasma. Alternatively, a platelet-rich plasma sample is preferred in another aspect. As it is used in the present invention, "platelet-rich plasma" refers to plasma that has been enriched with platelets and generally contains more than 300-350,000 platelets/pL. Various methods known in the state of the art can be used for obtaining platelet-rich plasma, including the methods described in Anitua and Andía, Puesta al Dia Publicaciones, 2000: 13-55; De Obarrio et al., Int J Periodontics Rest Dent 2000;20:487-497 and Camargo and Leckovics, J Clin Periodontol Res 2002;37:300-306; Okuda et al. J Periodontol 2003;74:849-857y Kawase et al. J Periodontol 2003;74:858-864). The various methods consist of obtaining blood in tubes with anticoagulant and subjecting them to different centrifugation conditions according to the different protocols, such that the blood separates into its basic components depending on density, selecting that fraction corresponding to the platelet-rich plasma. Commercial kits such as RegenPRP-Kit (RegenLab) can alternatively be used.

According to the invention, the blood plasma fraction to be analyzed can be obtained previously from a human blood sample (preferably human peripheral blood) already obtained by means of any of the standard blood fractionation processes, such as centrifugation-filtration fractionation for example. Nevertheless, even though the previous fractionation step is not necessary, to obtain the Raman and/or infrared spectra, the blood plasma fraction sample may possibly have to be prepared differently when its Raman spectrum is to be recorded with respect to when its IR spectrum is to be recorded.

In the recording conditions of the embodiments, the blood plasma fraction samples were prepared for recording their Raman spectrum following a process comprising:
a) completely evaporating to dryness a volume of a blood plasma fraction to obtain at least between 1 and 3 mg of a dry solid residue of said fraction, where the evaporation of said fraction volume is performed at a temperature comprised between 2 and 25°C, preferably between 15 and 25°C, because evaporation is obtained more quickly than at lower temperatures.
b) collecting the previous dry fraction and transferring it, by means of a conventional micromortar for FT-Raman spectroscopy, to a cylindrical aluminum pan with a semi-spherical hollow of 2 mm in diameter.

On the other hand, in the recording conditions of the embodiments, the blood plasma fraction samples were prepared for recording their infrared spectrum following a process comprising extending a volume of between 3 and 7 µL of a blood plasma fraction on a ZnSe crystal and leaving it to completely evaporate to dryness at a temperature comprised between 2 and 25°C, preferably between 15 and 25°C.

When the blood plasma fraction samples are prepared for recording their Raman or infrared spectrum, the evaporation to dryness of the volume of said fraction can be performed at a temperature comprised between 2 and 25°C without observing any alteration of the samples for the purpose of the present invention. Evaporation is preferably performed between 15 and 25°C, both limits being included, for the purpose of faster sample evaporation.

Generally and in a non-limiting manner, when a non-fractionated human blood sample is used initially, the blood fraction sample (preferably a blood plasma sample) suitable for Raman spectroscopic analysis of the present invention can be prepared prior to acquiring the spectroscopic data according to a process comprising the following steps:
a. obtaining said blood fraction (preferably the blood plasma fraction) from a previously obtained human peripheral blood sample by means of any of the known centrifugation-filtration blood fractionation processes normally used;
b. completely evaporating to dryness a volume of the previous fraction, approximately 40 µL, to obtain at least between 1 and 3 mg of a dry solid residue of said fraction, where the evaporation of said fraction volume is performed at relatively low temperatures, specifically in the temperature range comprised between 2 and 25°C, more preferably between 15 and 25°C, said limits being included;
c. collecting the previous dry fraction and transferring it, by means of a conventional micromortar for FT-Raman spectroscopy, to a cylindrical aluminum pan with a semi-spherical hollow of 2 mm in diameter.

When the method of the present invention also comprises an infrared spectroscopic analysis of the blood sample, preferably of the same blood plasma sample, in addition to the Raman spectroscopic analysis of the blood sample, the blood or plasma samples must be prepared under conditions suitable for recording their IR spectra prior to acquiring the spectroscopic data. By way of non-limiting example, when a non-fractionated human blood sample is used initially, the blood plasma fraction sample suitable for infrared spectroscopic analysis is prepared previously according to a process comprising the following steps:
a) obtaining said blood fraction (preferably the blood plasma fraction) from a previously obtained human peripheral blood sample by means of any of the known centrifugation-filtration blood fractionation processes normally used;
b) extending a volume of the fraction obtained in step h on a ZnSe crystal to obtain reliable spectra, which volume in the case of plasma is preferably between 3 and 7 µL, and leaving it to completely evaporate to dryness at a temperature comprised between 2 and 25°C, preferably between 15 and 25°C.

According to the invention, the "Raman spectral value" refers to a value obtained from the Raman spectral profile of one of the regions defined in R.1 to R.4, such as a Raman intensity ratio between the two bands of greater intensity in said region, a value of the area comprised between the two minimum values of said spectral region or the frequency value of one of the bands of said region. The authors of the invention have found the bands having the greatest differences between their intensities and the Global Deterioration Scale (GDS) to be the "bands approximating" or "the bands located around" 1671, 1658, 960, 938, 758, 743, 423, 409 cm⁻¹. The intensities of these bands or the areas enclosed under the spectral profile comprised between two of these bands of one and the same spectral region can therefore be suitable spectral values.

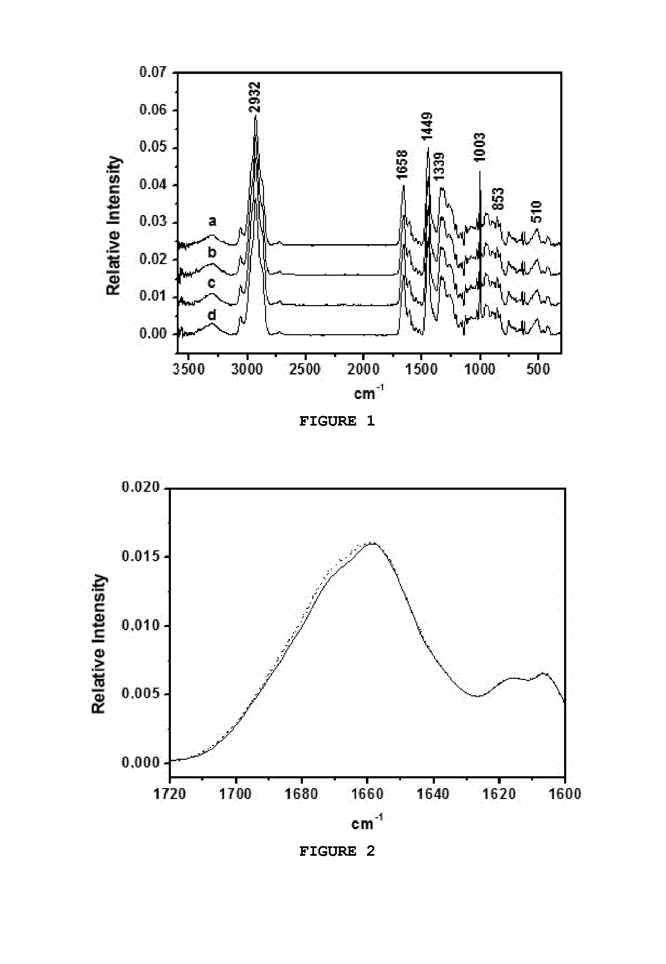

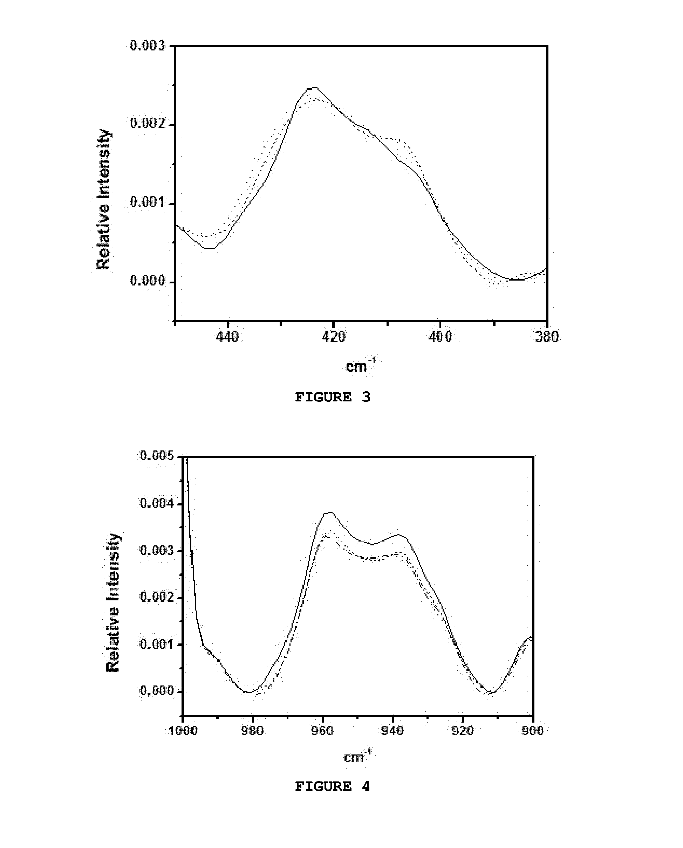

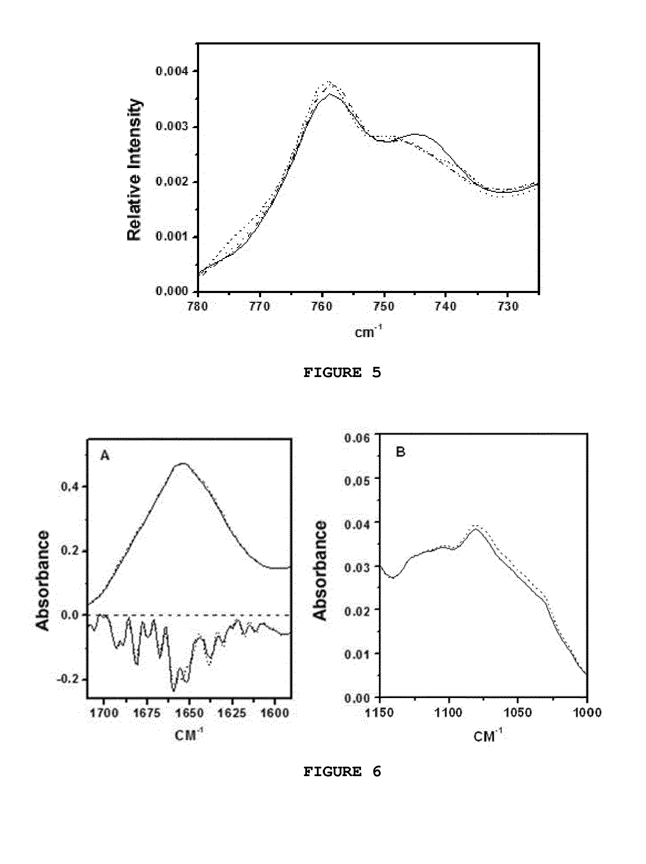

The biochemical-structural analysis of the mentioned spectroscopic values or parameters has been performed by means of characteristic spectra of the various types of biomolecules present in the blood (De Gelder et al., J. Raman Spectrosc. 38, 1133-1147 (2007); Socrates, Infrared and Raman Characteristic Group Frequencies (3rd ed.). John Wiley and Sons, Chichester, 2001). The highest Raman intensity at 1671 and 409 cm⁻¹ (Figures 2-3) in AD patients (preferably determined as a ratio between the intensities at 1671 and 1658 cm⁻¹ and as the ratio between the intensities at 409 and 423 cm⁻¹) can be attributed to the formation of β-sheet protein structure owing to considerations of group frequencies and by comparison with Raman spectra of Aβ-40 and Aβ-42 amyloid peptides which have been measured in this invention under the same spectral recording conditions as the blood samples. The reduction in the area between 980 and 910 cm⁻¹ in the course of the disease (Figure 4) can be interpreted in terms of a reduction in α-helix protein structure, and finally the change of the spectral profile between 760 and 740 cm⁻¹ (Figure 5) (determined as the frequency of the band in said range) can be assigned to an alteration in the tertiary structure of certain proteins which is clearly shown in this spectral range of tryptophan.

Similarly and according to the invention, the "infrared spectral value" refers to a value obtained from the infrared spectral profile of one of the regions defined in IR.1 and IR. 2, such as, among others for example, a percentage of the area of a component band of the spectral profile of said region, or the normalized area of the spectral profile of said region. As previously mentioned, in determining the protein structure associated with global cognitive deterioration in Alzheimer's disease, the spectral region comprised between 1600-1700 cm⁻¹ provides information referring to the content of total β-peptide structure of the blood sample to be analyzed, and more specifically the area of the spectral region comprised between 1623 and 1640 cm⁻¹. However, the spectral region comprised between 1000-1150 cm⁻¹ would be associated with markers of oxidative stress associated with AD, which are helpful in obtaining better discrimination of the blood sample to be analyzed in determining the protein structure associated with global cognitive deterioration in Alzheimer's disease.

In the scope of the present invention, the expression "reference spectral value of the Ri region, allowing the distinction between class I or II" refers to the cut-off value (associated with the highest sensitivity and specificity) obtained by means of the ROC curve for the spectral variable of said region Ri. When the classification of step c is carried out by means of multivariate analysis considering two or more spectral regions of those previously defined (R.1 to R.4 Raman regions and/or IR.1 and IR.2 infrared regions), the reference spectral values refer to the corresponding discriminant cut-off point (C), i.e., to the mean value of the centroids of the two groups I and II (*D_{I}* and *D_{II}*) ; each centroid being the discriminant score (discriminant function value) for the averages of each of the discriminant variables in each group. In other words, the centroid of a group is a point whose coordinates are the averages in the group of each of the discriminant variables. If the discriminant score Di for the sample i is such that the *Dᵢ*-*D_{I}*, difference in absolute value is less than the C-D_{I} difference in absolute value, said sample is considered to belong to group I, and it will otherwise belong to group II.

According to the present invention, suitable Raman spectral values can be the following:
- the ratio of Raman intensities obtained around 1671 cm⁻¹ and around 1658 cm⁻¹;
- the ratio of Raman intensities obtained around 409 cm⁻¹ and around 423 cm⁻¹;
- the area of the Raman spectral profile comprised between 910 and 980 cm⁻¹;
- the ratio of Raman intensities obtained around 758 cm⁻¹ and around 743 cm⁻¹ or the frequency of band comprised in the Raman spectrum region between 740 and 750 cm⁻¹.

The infrared spectral values suitable for being used in the present invention are, among others:
- the percentage of the area of β-sheet protein structure measured preferably in the amide I infrared region;
- the area of the infrared spectral profile between 1150 and 1000 cm⁻¹ relating to oxidative stress.

It is also possible to use two or more of the previous spectral values together to obtain better results.

In a preferred embodiment of the methods of the invention, when said methods comprise steps a-c of the first method of the invention, as well as when they comprise steps a-c of the second method of the invention in addition to steps a-c of the first method of the invention, the spectral value of the Raman spectrum region defined in R.1 comprises the ratio of Raman intensities obtained around 1671 cm⁻¹ and around 1658 cm⁻¹.

In the scope of the present invention, the expression "band around a frequency value vᵢ expressed in cm⁻¹" refers to its (Raman or IR) vibrational spectrum band that is obtained with maximum intensity in the frequency range determined by vᵢ ± X cm⁻¹, wherein X is a variable frequency value that is at least 1, at least 2, at least 3, at least 4, at least 5, at least 6, at least 7, at least 8, at least 9, at least 10 or greater. In a preferred embodiment, X has a value of 2. Therefore a band around a frequency value of 1658 cm⁻¹, for example, refers to the maximum band that is obtained in the 1656 to 1660 cm⁻¹ range, expressed in frequency values. In the present specification, said term can also be referred to as "band approximating a frequency value expressed in cm⁻¹". The increase of ± X cm⁻¹ in a band frequency is indicated to define a frequency range amplitude of one and the same band which can be measured in various spectrometers differing in the frequency measurement accuracy. Therefore, it must be understood that the frequency values vᵢ appearing in this specification can vary by ± X cm⁻¹ with respect to said frequency value depending on the spectrometer used.

Accordingly, the term "Raman intensity obtained around a frequency value vᵢ expressed in_cm⁻¹"is understood to refer to the intensity value of the Raman spectrum obtained at the maximum of the band appearing in the frequency range determined by vᵢ ± X cm⁻¹ In the event that X has a value of 2, a Raman intensity obtained around a frequency value of 1671 cm⁻¹ corresponds to the intensity value of the maximum obtained in the frequency range comprised between 1669 cm⁻¹ and 1673 cm⁻¹. Similarly, the term "IR intensity obtained around a frequency value vᵢ expressed in cm⁻¹" refers to the intensity value of the IR spectrum obtained at the maximum of the band appearing in the frequency range determined by vᵢ ± X cm⁻¹.

In another preferred embodiment of the methods of the invention, when said methods comprise steps a-c of the first method of the invention, as well as when they comprise steps a-c of the second method of the invention in addition to steps a-c of the first method of the invention, the spectral value of the Raman spectrum region defined in R.4 comprises the ratio of Raman intensities obtained around 409 cm⁻¹ and around 423 cm⁻¹. And in a more preferred embodiment of the methods, the spectral value of the Raman spectrum region defined in R.4 comprises the ratio of Raman intensities obtained around 409 cm⁻¹ and around 423 cm⁻¹, and the spectral value of the Raman spectrum region defined in R.1 comprises the ratio of Raman intensities obtained at 1671 cm-¹ and 1658 cm⁻¹.

In another preferred embodiment of the methods of the invention or any of their previous embodiments, when said method comprises steps a-c of the first method of the invention, as well as when it comprises steps a-c of the second method of the invention in addition to steps a-c of the first method, the spectral value of the Raman spectrum region defined in R.2 comprises the area of the Raman spectral profile comprised between 910 and 980 cm⁻¹.

In another preferred embodiment of the methods of the invention or any of their previous embodiments, when the methods comprise steps a-c of the first method of the invention, as well as when they comprise steps a-c of the second method of the invention in addition to steps a-c of the first method of the invention, the spectral value of the Raman spectrum region defined in R.3 comprises the ratio of Raman intensities obtained at the maximum of the band around 758 cm⁻¹ and at the maximum of the band located in the 740-750 cm⁻¹ region, or it comprises the frequency of the band comprised in the Raman spectrum region between 740 and 750 cm⁻¹.

In a preferred embodiment of the methods, when said methods comprise steps a-c of the second method of the invention, the spectral value of the infrared spectrum region defined in IR.1 comprises the percentage of the area of the spectral profile between 1640 and 1623 cm⁻¹ expressed in second derivatives.

In another preferred embodiment of the methods, when said methods comprise steps a-c of the second method of the invention, the spectral value of the infrared spectrum region defined in IR.2 comprises the percentage of the area of the spectral profile between 1150 and 1000 cm⁻¹. And in a more preferred embodiment of the methods comprising steps a-c of the second method of the invention, the spectral value of the infrared spectrum region defined in IR.2 comprises the percentage of the area of the spectral profile between 1150 and 1000 cm⁻¹, and the spectral value of the infrared spectrum region defined in IR.1 further comprises the percentage of the area of the spectral profile between 1640 and 1623 cm⁻¹ expressed in second derivatives.

In a preferred embodiment, the percentage of the area of the 1640 and 1623 cm⁻¹ region is calculated by multiplying the quotient of the area of the 1640-1623 cm⁻¹ profile and the area of the amide I band between 1670 and 1623 cm⁻¹ by 100.

In a preferred embodiment, the percentage of the area of the region is calculated by multiplying the quotient of the spectral profile area between 1150 and 1000 cm⁻¹ and the area of the spectral profile between 3010 and 2800 cm⁻¹ by 100.

According to the invention, the methods defined above can be carried out such that step b of the first method comprises obtaining a spectral value of the Raman spectrum region defined in R.1, being preferred when said spectral value of the region between 1600 and 1700 cm⁻¹ is the ratio of Raman intensities obtained around 1671 cm⁻¹ and around 1658 cm⁻¹, or in other words, the height of the β-protein structure band around 1671 cm⁻¹ normalized with respect to the height of the amide I band around 1658 cm⁻¹. It is therefore a relative Raman intensity that is proportional to the β-protein structure percentage of the blood sample obtained to be analyzed. An alternative process would be to fit the amide I spectral profile to a sum of Gaussian functions, determining the β structure percentage as a percentage of the area. However, given that β structure Raman signal around 1671 cm⁻¹ is weak and the fit is to a certain extent subjective because it depends on the initial parameters entered, the result may not be unique, and this conventional spectral profiles fitting methodology has in fact been unsuccessfully applied in this work.

Said relative Raman intensity proportional to the β-peptide structure percentage of the test blood sample obtained can serve as a discriminant value for classifying said sample in class I or in class II, as they were defined above, by comparison with a reference numerical parameter, serving as a limit value between both classes. This limit reference numerical parameter is the result of experimental statistical studies. In a particular embodiment, said relative Raman intensity is equal to or less than 0.85, the test blood sample obtained belongs to a class I (without cognitive deterioration), and if I is greater than 0.85 said blood sample belongs to a class II (cognitive deterioration associated with Alzheimer's disease). According to this classification of the Raman spectrum, a result with a sensitivity and specificity around 70% approximately is obtained. This sensitivity relates to the probability that a class II sample subjected to analysis by this method leads to a class II positive result, and percentage-wise it is calculated by multiplying by one hundred the quotient of the number of hits of class II samples and the total number of class II samples analyzed. Concerning specificity, it relates to the probability that a class I sample subjected to said analysis leads to a positive class I result, and percentage-wise it is calculated by multiplying the quotient of the number of hits of class I samples and the total number of class I samples analyzed by one hundred.

A more preferred possibility of carrying out the methods of the invention in any of its variants or embodiments, because more sensitive and specific results are achieved, consists of step b of said first method, in addition to obtaining a spectral value of the Raman spectrum region defined in R.1, further obtaining the spectral values of the Raman spectrum regions defined in R.2, R.3 and R.4, where said spectral values can be any of those defined above in the present description. In this case, in step c of said method, the classification is obtained by multivariate analysis comparison (e.g. discriminant analysis) of the previous spectral values with a class I reference spectral value and with a class II reference spectral value, where said reference values are obviously analogous, and therefore comparable, to those obtained in step b, being able to have been previously determined (e.g. forming part of a spectroscopic value database like the one defined above in the specification, or it can be obtained previously and stored to be included in successive studies with the method of the invention, etc.). The important effect that introducing the value in region R.4 (ratio of intensities 409/423 cm⁻¹) has on sensitivity and specificity in classifying samples by means of discriminant analysis should be mentioned. When it is added to the other spectral values defined for regions R.1, R.2 and R.3, the sensitivity and specificity increase from approximately 80% to more than 90% (see Examples 4 and 5).

Another possibility of carrying the methods of the invention in any of its variants or embodiments whereby even better results are obtained, consists of said methods comprising: in step b, obtaining the spectral values of the Raman spectrum regions defined in R.1, R.2, R.3 and R.4, obtaining the spectral values of the infrared spectrum regions defined in IR.1 and IR.2; and in step c, classifying by means of multivariate analysis comparison of said spectral values (Raman and infrared) with classes I and II reference Raman spectral values, and with classes I and II reference infrared spectral values, where said reference spectral values are comparable to those obtained in step b. The effect of improvement in classifying samples which incorporate infrared parameters in the discriminant analysis should also be pointed out (see example 6). According to the two preceding possibilities for carrying out the method of the invention, it is preferable for the class II reference spectral value (whether the reference Raman spectral value or values alone or in combination with the reference infrared spectral value or values) to comprise at least one reference spectral value of the following sub-classes:
II-a. spectral value corresponding to a blood sample containing the protein structure associated with mild cognitive deterioration in Alzheimer's disease, with a value of GDS-3 on Reisberg's global cognitive deterioration scale.
II-b. spectral value corresponding to a blood sample containing the protein structure associated with mild cognitive deterioration in Alzheimer's disease, with a value of GDS-4 on Reisberg's global cognitive deterioration scale.
II-c. spectral value corresponding to a blood sample containing the protein structure associated with mild cognitive deterioration in Alzheimer's disease, with a value of GDS-5 on Reisberg's global cognitive deterioration scale.
II-d. spectral value corresponding to a blood sample containing the protein structure associated with mild cognitive deterioration in Alzheimer's disease, with a value of GDS-6 on Reisberg's global cognitive deterioration scale.
II-e. spectral value corresponding to a blood sample containing the protein structure associated with mild cognitive deterioration in Alzheimer's disease, with a value of GDS-7 on Reisberg's global cognitive deterioration scale.

In a preferred embodiment of the methods of the invention, the reference spectral value (including any reference Raman spectral value and any reference infrared spectral value) is obtained under the same conditions described in steps b of the first or second method of the invention, from reference blood samples as they are defined in class I and in class II, or from reference blood samples as they are defined in class I, in sub-class II-a, in sub-class II-b, in sub-class II-c, in sub-class II-d and in sub-class II-e.

In a preferred embodiment, when the method comprises the classification of step c by means of multivariate analysis, said method of analysis is a method of discriminant analysis using the following as independent variables:
- the spectral value or values obtained in step b and the reference spectral values, i.e., the height ratios 1671/1658, 758/743, 409/423 cm⁻¹, frequency of the band in the range 740-750 cm⁻¹, and area of the spectral profile between 980-910 cm⁻¹ normalized with respect to the area of the amide I band, of the spectrum of the first blood sample obtained, and the height ratios 1671/1658, 758/743, 409/423 cm⁻¹, frequency of the band in the range 740-750 cm⁻¹, and area of the spectral profile between 980-910 cm⁻¹, normalized with respect to the area of the amide I band, of the spectrum of each reference blood sample obtained;
   and the following as a grouping variable:
- the variables indicative of classes I or II to which each of the spectroscopic reference values belongs.

Without limiting the scope of the invention and by way of example, the process of the invention by means of using the Raman spectroscopy can comprise the following steps:
1) Obtaining a peripheral blood plasma fraction by means of centrifugation-filtration.
2) Drying 50 µL of plasma at room temperature for a Raman spectra measurement in solid state.
3) Raman spectra measurement and subsequent mathematical processing of spectra for: (a) determining the aforementioned spectroscopic parameters; and (b) performing discriminant analysis for classifying samples in the mentioned cognitive state classes I and II.

In the same non-limiting manner, by way of example the process of the invention by means of the combined use of the Raman and infrared vibrational spectroscopies can comprise the following steps:
1) Obtaining a peripheral blood plasma fraction by means of centrifugation-filtration.
2) Drying 50 µL of plasma at room temperature for a Raman spectra measurement in solid state.
3) Drying a volume between 3 and 7 µL of plasma extended on a circular ZnSe crystal for infrared spectroscopy at room temperature
4) Raman and infrared spectra measurement and subsequent mathematical processing of spectra for: (a) determining the aforementioned spectroscopic parameters; and (b) performing discriminant analysis for classifying samples in the mentioned cognitive state classes I and II.

An object of the present invention is the method based on discriminant analysis to obtain an indication of the presence or absence of a condition of Alzheimer's disease in a blood sample obtained, based on classifying said sample in the classes or sub-classes defined in this specification. Therefore, if a blood sample analyzed by this method is classified as class I, it is indicative of a condition of the absence of AD. In the same manner, if said spectroscopic analysis classifies a blood sample as class II, or any of its sub-classes II-a, II-b, II-c, II-d and II-e, said analysis is indicative of a condition of the presence of AD. Therefore, the methods of the present invention can be used for clinical purposes to assess an individual of interest for diagnostic purposes and/or to assess the individual's degree of development in Alzheimer's disease, from a blood sample previously obtained from said individual.

Accordingly, another aspect of the invention relates to the method of the present invention defined above to obtain an indication of the presence or absence of a condition of Alzheimer's disease from the analysis of a human blood sample, being preferred that method comprising the comparison of step c by means of multivariate analysis. In other words, this second aspect relates to the use of the method of the present invention to obtain an indication of the presence or absence of a condition of Alzheimer's disease from the analysis of an already obtained human blood sample.

In a another aspect, the invention refers to the method of the present invention comprising the multivariate analysis comparison of step c to obtain an indication of the degree of global cognitive deterioration associated with Alzheimer's disease from the analysis of a human blood sample.

In another aspect, the invention relates to a diagnostic method (first diagnostic method) for diagnosing Alzheimer's disease or for determining the global cognitive deterioration associated with Alzheimer's disease in a subject comprising the steps of:
a) measuring a Raman spectrum of a plasma sample from said subject obtaining at least one Raman band selected from the group consisting of a Raman band comprising vibrations specific to β-protein structure, a Raman band of amyloid peptides comprising vibrations specific to angular deformation of the peptide bond, a Raman band comprising vibrations specific to α-helix protein structure and a Raman band comprising vibrations specific to tertiary protein structure with tryptophan residues and
b) comparing the Raman spectrum obtained in step (a) with the spectrum of a reference sample
wherein a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum and/or a Raman spectrum variation indicative of an increase in the vibrations specific to tryptophan residues in a tertiary protein structure with respect to the reference spectrum is indicative of the patient having Alzheimer's disease or of the patient suffering cognitive deterioration associated with Alzheimer's disease.

As it is used herein, "diagnostic method" refers to evaluating the probability according to which a subject suffers a disease (in this case, Alzheimer's disease). As persons skilled in the art will understand, such evaluation may not be correct for 100% of the subjects to be diagnosed, even though it preferably is. The term, however, requires being able to identify a statistically significant portion of the subjects as suffering said disease or having a predisposition to suffer said disease. The person skilled in the art can determine if a portion is statistically significant using various well-known statistical evaluation tools, for example, by means of determining confidence intervals, determining p value, Student's t-test, Mann-Whitney test, etc. Information and details about said tools can be found in Dowdy and Wearden, Statistics for Research, John Wiley & Sons, New York 1983. The preferred confidence intervals are at least 50%, at least 60%, at least 70%, at least 80%, at least 90% at least 95%. The p values are preferably 0.05, 0.025, 0.001 or less.

"Raman band comprising vibrations specific to β-protein structure" is understood as the representation of the inelastic scattering of a photon generated by β-protein structures when a monochromatic light beam strikes them. "β-protein structure" refers to the structure formed by the parallel positioning of two amino acid chains in the same protein, where the N-H groups of one of the chains form hydrogen bonds with the C=0 groups of the opposite chain. There are two types: parallel chains (those in which both go from an amino to carboxyl group) and antiparallel chains (those which go from amino to carboxyl and another from carboxyl to amino). The hydrogen bridge between N-H and C=O of adjacent chains has a length (H ... 0) of ~0.17- 0.18 nm and an angle (N-H with C=0) of ~5-10°, except for antiparallel beta sheets in which both values are greater.

"Raman band of amyloid peptides comprising vibrations specific to angular deformation of the peptide bond" according to the present invention is understood as the representation of the inelastic scattering of a photon generated by the angular deformation of the peptide bond when a monochromatic light beam strikes them. As it is used in the present invention, "angular deformation of the peptide bond" refers to the movement of an atom outside the axis of the bond. There are various types of deformation according to the atom that is being moved, but generally they are rotations around the NC bond, leading to deviations of the perfect (0°) or trans (180°) cis ω angle.

As it is used in the present invention, "Raman band comprising vibrations specific to α-helix protein structure" refers to the representation of the inelastic scattering of a photon generated by the structure of α-helix protein when a monochromatic light beam strikes them. As it is used in the present invention, "α-helix protein structure" refers to the structure formed by the arrangement in a right-handed helical structure of about 3.6 amino acids per turn. Each amino acid entails a rotation of about 100° in the helix, and the Cα of two contiguous amino acids are separated by 1.5 Å**.** The helix is tightly packed, such that there is virtually no free space in the helix. All the amino acid side chains are arranged towards the outside of the helix. One of the most defining features of an α-helix is the presence of a hydrogen bridge between the N-H of the peptide bond and the C=0 of the peptide bond i + 4 (i→i+4). For a standard α-helix this hydrogen bridge has a length (H ... 0) of ~0.18 nm and an angle (N-H with C=0) of ~3°.

According to the present invention, "Raman band comprising vibrations specific to tertiary protein structure with tryptophan residues" refers to the representation of the inelastic scattering of a photon generated by tryptophan residues in a tertiary protein structure when a monochromatic light beam strikes them. As it is used in the present invention, "tryptophan" refers to a CAS no. 73-22-3 amino acid classified among hydrophobic, apolar amino acids.

In a particular embodiment, the diagnostic method comprises obtaining any one combination of two Raman bands selected from the group consisting of:
- a Raman band comprising vibrations specific to β-protein structure and a Raman band of amyloid peptides comprising vibrations specific to angular deformation of the peptide bond;
- a Raman band comprising vibrations specific to β-protein structure and a Raman band comprising vibrations specific to α-helix protein structure;
- a Raman band comprising vibrations specific to β-protein structure and a Raman band comprising vibrations specific to tertiary protein structure with tryptophan residues;
- a Raman band of amyloid peptides comprising vibrations specific to angular deformation of the peptide bond and a Raman band comprising vibrations specific to α-helix protein structure;
- a Raman band of amyloid peptides comprising vibrations specific to angular deformation of the peptide bond and a Raman band comprising vibrations specific to tertiary protein structure with tryptophan residues;
- a Raman band comprising vibrations specific to α-helix protein structure and a Raman band comprising vibrations specific to tertiary protein structure with tryptophan residues.

In another particular embodiment, the diagnostic method of the invention comprises obtaining any one combination of three Raman bands selected from the group consisting of:
- a Raman band comprising vibrations specific to β-protein structure, a Raman band of amyloid peptides comprising vibrations specific to angular deformation of the peptide bond and a Raman band comprising vibrations specific to α-helix protein structure;
- a Raman band comprising vibrations specific to β-protein structure, a Raman band of amyloid peptides comprising vibrations specific to angular deformation of the peptide bond and a Raman band comprising vibrations specific to tertiary protein structure with tryptophan residues;
- a Raman band comprising vibrations specific to β-protein structure, a Raman band comprising vibrations specific to α-helix protein structure and a Raman band comprising vibrations specific to tertiary protein structure with tryptophan residues;
- a Raman band of amyloid peptides comprising vibrations specific to angular deformation of the peptide bond, a Raman band comprising vibrations specific to α-helix protein structure and a Raman band comprising vibrations specific to tertiary protein structure with tryptophan residues.

In another particular embodiment, the diagnostic method of the invention comprises obtaining a Raman band comprising vibrations specific to β-protein structure, a Raman band of amyloid peptides comprising vibrations specific to angular deformation of the peptide bond, a Raman band comprising vibrations specific to α-helix protein structure and a Raman band comprising vibrations specific to tertiary protein structure with tryptophan residues.

In a particular embodiment of the diagnostic method of the invention, the Raman band comprising vibrations specific to beta sheet protein is the band around 1671 cm⁻¹. In another particular embodiment, the Raman band comprising vibrations specific to angular deformation of the peptide bond is the band around 409 cm⁻¹. In another particular embodiment, the Raman band comprising vibrations specific to α-helix protein structure is defined by the spectral profile between 980 and 910 cm⁻¹. In another particular embodiment, the Raman band comprising vibrations specific to tryptophan residues in a tertiary protein structure is the band around 758 cm⁻¹ and/or the band around the 740-750 cm⁻¹ region.

In a particular embodiment of the diagnostic method of the invention, the Raman spectrum variation indicative of an increase in intensities specific to β-protein structure with respect to the reference spectrum is an increase in the ratio between the intensities located around 1671 cm⁻¹ and 1658 cm⁻¹. In another particular embodiment, the Raman spectrum variation indicative of an increase in intensities specific to tryptophan residues in a tertiary protein structure with respect to the reference spectrum is selected from the group consisting of an increase in the intensity of the maximum of the band located around 758 cm⁻¹ and an increase in the frequency of the band located in the spectral range of 740-750 cm⁻¹. In another particular embodiment, the Raman spectrum variation indicative of a reduction in intensities specific to α-helix protein structure with respect to the reference spectrum is a reduction in the area of the spectral profile between 980-910 cm⁻¹. In another particular embodiment, the Raman spectrum variation indicative of an increase in vibrations specific to angular deformation of the peptide bond with respect to the reference spectrum is an increase in the ratio between the intensities of the bands located around 409 cm⁻¹ and 423 cm⁻¹.

In a particular embodiment of the diagnostic method of the invention, the Raman spectrum is recorded using a near-infrared laser excitation line.

In another aspect, the invention relates to a diagnostic method (second diagnostic method) for diagnosing Alzheimer's disease in a subject comprising the steps of:
a) measuring an IR spectrum of a plasma sample from said subject obtaining at least one band specific to the presence of β-protein structure and/or in at least one specific band indicative of the presence of compounds generated during oxidative stress and
b) comparing the IR spectrum obtained in step (a) with the spectrum of a reference sample
wherein an IR spectrum variation indicative of an increase in β-protein structure with respect to the reference spectrum and/or an IR spectrum variation indicative of an increase in the concentration of compounds generated in the sample during oxidative stress with respect to the reference spectrum is indicative of the patient having Alzheimer's disease.

In relation to an IR spectrum, "band specific to the presence of β-protein structure" refers to the representation of the infrared radiation absorption by a β-protein structure. The term "β-protein structure" has been defined above in relation to the first diagnostic method.

As it is used in the present invention, "band specific to the presence of compounds generated during oxidative stress" refers to the representation of the infrared radiation absorption by the compounds generated in a situation of disequilibrium between the production of reactive oxygen species and the capacity of the biological system to detoxify said reactive oxygen species when an infrared beam strikes them. As it is used in the present invention, "reactive oxygen species" refers to oxygen ions, free radicals and both organic and inorganic peroxides, which are highly reactive due to the presence of a layer of unpaired valence electrons.

In a particular embodiment of the second diagnostic method of the invention, said method comprises measuring an IR spectrum of a plasma sample from said subject obtaining a band specific to the presence of β-protein structure and a specific band indicative of the presence of compounds generated during oxidative stress.

In a particular embodiment of the second diagnostic method of the invention, the band specific to the presence of β-protein structure is the band around 1640-1623 cm⁻¹. In another particular embodiment, the IR spectrum variation indicative of an increase in β-protein structure with respect to the reference spectrum is an increase in the spectral profile area in the region between 1640-1620 cm⁻¹, more particularly said increase is determined as a percentage of the area in the region between 1640-1620 cm⁻¹ of the spectrum expressed in second derivative. To calculate the percentage of the area of the 1640 and 1623 cm⁻¹ region, the quotient between the area of the 1640-1623 cm⁻¹ profile and the area of the amide I band between 1670 and 1623 cm⁻¹ is multiplied by 100.

In another particular embodiment, the band specific to the presence of compounds generated during oxidative stress is the band between 1150 cm⁻¹ and 1000 cm⁻¹. In another particular embodiment of the second diagnostic method of the invention, the IR spectrum variation indicative of an increase in the concentration of compounds generated in the sample during oxidative stress with respect to the reference spectrum is an increase in the spectral profile area in the region between 1150 cm⁻¹ and 1000 cm⁻¹. In a particular additional embodiment said increase is normalized with respect to the area in the region between 3010-2800 cm⁻¹. To calculate the percentage of spectral profile area between 1150 and 1000 cm⁻¹, the value 100 is used as the area of the spectral profile between 3010 and 2800 cm⁻¹.

In another aspect, the first diagnostic method of the invention additionally comprises the steps of the second method of the invention applied to a sample from the same subject. In a particular embodiment, the first diagnostic method of the invention comprises obtaining at least one Raman band selected from the group consisting of a Raman band comprising vibrations specific to β-protein structure, a Raman band comprising vibrations specific to angular deformation of the peptide bond, a Raman band comprising vibrations specific to α-helix protein structure and a Raman band comprising vibrations specific to tertiary protein structure with tryptophan residues
and
wherein the second diagnostic method of the invention comprises measuring an IR spectrum obtaining a band specific to the presence of β-protein structure and a specific band indicative of the presence of compounds generated during oxidative stress
wherein
a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum, a Raman spectrum variation indicative of an increase in intensities specific to tryptophan residues in a protein structure with respect to the reference spectrum, an IR spectrum variation indicative of an increase in beta sheet protein structure with respect to the reference spectrum and an IR spectrum variation indicative of an increase in the concentration of compounds generated in the sample during oxidative stress with respect to the reference spectrum is indicative of the patient having Alzheimer's disease.

Therefore, in preferred embodiments, the diagnostic method of the invention comprises determining two spectral bands selected from the group consisting of:
- a Raman band comprising vibrations specific to β-protein structure and an IR band specific to the presence of β-protein structure,
- a Raman band comprising vibrations specific to β-protein structure and a specific IR band indicative of the presence of compounds generated during oxidative stress,
- a Raman band of amyloid peptides comprising vibrations specific to angular deformation of the peptide bond and an IR band specific to the presence of β-protein structure,
- a Raman band of amyloid peptides comprising vibrations specific to angular deformation of the peptide bond and a specific IR band indicative of the presence of compounds generated during oxidative stress,
- a Raman band comprising vibrations specific to α-helix protein structure and an IR band specific to the presence of β-protein structure,
- a Raman band comprising vibrations specific to α-helix protein structure and a specific IR band indicative of the presence of compounds generated during oxidative stress,
- a Raman band comprising vibrations specific to tertiary protein structure with tryptophan residues and an IR band specific to the presence of β-protein structure,
- a Raman band comprising vibrations specific to tertiary protein structure with tryptophan residues and a specific IR band indicative of the presence of compounds generated during oxidative stress,

In preferred embodiments, the diagnostic method of the invention comprises determining three spectral bands selected from the group consisting of:
- a Raman band comprising vibrations specific to β-protein structure, a Raman band of amyloid peptides comprising vibrations specific to angular deformation of the peptide bond and an IR band specific to the presence of β-protein structure,
- a Raman band comprising vibrations specific to β-protein structure, a Raman band of amyloid peptides comprising vibrations specific to angular deformation of the peptide bond and a specific IR band indicative of the presence of compounds generated during oxidative stress,
- a Raman band comprising vibrations specific to β-protein structure, a Raman band comprising vibrations specific to α-helix protein structure and an IR band specific to the presence of β-protein structure,
- a Raman band comprising vibrations specific to β-protein structure, a Raman band comprising vibrations specific to α-helix protein structure and a specific IR band indicative of the presence of compounds generated during oxidative stress,
- a Raman band comprising vibrations specific to β-protein structure, a Raman band comprising vibrations specific to tertiary protein structure with tryptophan residues and an IR band specific to the presence of β-protein structure,
- a Raman band comprising vibrations specific to β-protein structure, a Raman band comprising vibrations specific to tertiary protein structure with tryptophan residues and a specific IR band indicative of the presence of compounds generated during oxidative stress,
- a Raman band comprising vibrations specific to β-protein structure, an IR band specific to the presence of β-protein structure and a specific IR band indicative of the presence of compounds generated during oxidative stress,
- a Raman band of amyloid peptides comprising vibrations specific to angular deformation of the peptide bond, a Raman band comprising vibrations specific to α-helix protein structure and an IR band specific to the presence of β-protein structure,
- a Raman band of amyloid peptides comprising vibrations specific to angular deformation of the peptide bond, a Raman band comprising vibrations specific to α-helix protein structure and a specific IR band indicative of the presence of compounds generated during oxidative stress,
- a Raman band of amyloid peptides comprising vibrations specific to angular deformation of the peptide bond, a Raman band comprising vibrations specific to tertiary protein structure with tryptophan residues and an IR band specific to the presence of β-protein structure,
- a Raman band of amyloid peptides comprising vibrations specific to angular deformation of the peptide bond, a Raman band comprising vibrations specific to tertiary protein structure with tryptophan residues and a specific IR band indicative of the presence of compounds generated during oxidative stress,
- a Raman band of amyloid peptides comprising vibrations specific to angular deformation of the peptide bond, an IR band specific to the presence of β-protein structure and a specific IR band indicative of the presence of compounds generated during oxidative stress,
- a Raman band comprising vibrations specific to α-helix protein structure, a Raman band comprising vibrations specific to tertiary protein structure with tryptophan residues and an IR band specific to the presence of β-protein structure,
- a Raman band comprising vibrations specific to α-helix protein structure, a Raman band comprising vibrations specific to tertiary protein structure with tryptophan residues and a specific IR band indicative of the presence of compounds generated during oxidative stress
- a Raman band comprising vibrations specific to α-helix protein structure, an IR band specific to the presence of β-protein structure and a specific IR band indicative of the presence of compounds generated during oxidative stress,
- a Raman band comprising vibrations specific to tertiary protein structure with tryptophan residues, an IR band specific to the presence of β-protein structure and a specific IR band indicative of the presence of compounds generated during oxidative stress.

In preferred embodiments, the diagnostic method of the invention comprises determining three spectral bands selected from the group consisting of:
- a Raman band comprising vibrations specific to β-protein structure, a Raman band of amyloid peptides comprising vibrations specific to angular deformation of the peptide bond, a Raman band comprising vibrations specific to α-helix protein structure and an IR band specific to the presence of β-protein structure,
- a Raman band comprising vibrations specific to β-protein structure, a Raman band of amyloid peptides comprising vibrations specific to angular deformation of the peptide bond, a Raman band comprising vibrations specific to α-helix protein structure and a specific IR band indicative of the presence of compounds generated during oxidative stress,
- a Raman band comprising vibrations specific to β-protein structure, a Raman band of amyloid peptides comprising vibrations specific to angular deformation of the peptide bond, a Raman band comprising vibrations specific to tertiary protein structure with tryptophan residues and an IR band specific to the presence of β-protein structure,
- a Raman band comprising vibrations specific to β-protein structure, a Raman band of amyloid peptides comprising vibrations specific to angular deformation of the peptide bond, a Raman band comprising vibrations specific to tertiary protein structure with tryptophan residues and a specific IR band indicative of the presence of compounds generated during oxidative stress,
- a Raman band comprising vibrations specific to β-protein structure, a Raman band of amyloid peptides comprising vibrations specific to angular deformation of the peptide bond, an IR band specific to the presence of β-protein structure and a specific IR band indicative of the presence of compounds generated during oxidative stress,
- a Raman band comprising vibrations specific to β-protein structure, a Raman band comprising vibrations specific to α-helix protein structure, a Raman band comprising vibrations specific to tertiary protein structure with tryptophan residues and an IR band specific to the presence of β-protein structure,
- a Raman band comprising vibrations specific to β-protein structure, a Raman band comprising vibrations specific to α-helix protein structure, a Raman band comprising vibrations specific to tertiary protein structure with tryptophan residues and a specific IR band indicative of the presence of compounds generated during oxidative stress,
- a Raman band comprising vibrations specific to β-protein structure, a Raman band comprising vibrations specific to α-helix protein structure, an IR band specific to the presence of β-protein structure and a specific IR band indicative of the presence of compounds generated during oxidative stress,
- a Raman band comprising vibrations specific to β-protein structure, a Raman band comprising vibrations specific to tertiary protein structure with tryptophan residues, an IR band specific to the presence of β-protein structure and a specific IR band indicative of the presence of compounds generated during oxidative stress,
- a Raman band of amyloid peptides comprising vibrations specific to angular deformation of the peptide bond, a Raman band comprising vibrations specific to α-helix protein structure, a Raman band comprising vibrations specific to tertiary protein structure with tryptophan residues and an IR band specific to the presence of β-protein structure,
- a Raman band of amyloid peptides comprising vibrations specific to angular deformation of the peptide bond, a Raman band comprising vibrations specific to α-helix protein structure, a Raman band comprising vibrations specific to tertiary protein structure with tryptophan residues and a specific IR band indicative of the presence of compounds generated during oxidative stress,
- a Raman band of amyloid peptides comprising vibrations specific to angular deformation of the peptide bond, a Raman band comprising vibrations specific to α-helix protein structure, an IR band specific to the presence of β-protein structure, a specific IR band indicative of the presence of compounds generated during oxidative stress,
- a Raman band of amyloid peptides comprising vibrations specific to angular deformation of the peptide bond, a Raman band comprising vibrations specific to tertiary protein structure with tryptophan residues, an IR band specific to the presence of β-protein structure and a specific IR band indicative of the presence of compounds generated during oxidative stress,
- a Raman band comprising vibrations specific to α-helix protein structure, a Raman band comprising vibrations specific to tertiary protein structure with tryptophan residues, an IR band specific to the presence of β-protein structure and a specific IR band indicative of the presence of compounds generated during oxidative stress.

In preferred embodiments, the diagnostic method of the invention comprises determining five spectral bands selected from the group consisting of:
- a Raman band comprising vibrations specific to β-protein structure, a Raman band of amyloid peptides comprising vibrations specific to angular deformation of the peptide bond, a Raman band comprising vibrations specific to α-helix protein structure, a Raman band comprising vibrations specific to tertiary protein structure with tryptophan residues and an IR band specific to the presence of β-protein structure,
- a Raman band comprising vibrations specific to β-protein structure, a Raman band of amyloid peptides comprising vibrations specific to angular deformation of the peptide bond, a Raman band comprising vibrations specific to α-helix protein structure, a Raman band comprising vibrations specific to tertiary protein structure with tryptophan residues and a specific IR band indicative of the presence of compounds generated during oxidative stress,
- a Raman band comprising vibrations specific to β-protein structure, a Raman band of amyloid peptides comprising vibrations specific to angular deformation of the peptide bond, a Raman band comprising vibrations specific to α-helix protein structure, an IR band specific to the presence of β-protein structure and a specific IR band indicative of the presence of compounds generated during oxidative stress,
- a Raman band comprising vibrations specific to β-protein structure, a Raman band of amyloid peptides comprising vibrations specific to angular deformation of the peptide bond, a Raman band comprising vibrations specific to tertiary protein structure with tryptophan residues, an IR band specific to the presence of β-protein structure and a specific IR band indicative of the presence of compounds generated during oxidative stress
- a Raman band comprising vibrations specific to β-protein structure, a Raman band comprising vibrations specific to α-helix protein structure, a Raman band comprising vibrations specific to tertiary protein structure with tryptophan residues, an IR band specific to the presence of β-protein structure and a specific IR band indicative of the presence of compounds generated during oxidative stress,
- a Raman band of amyloid peptides comprising vibrations specific to angular deformation of the peptide bond, a Raman band comprising vibrations specific to α-helix protein structure, a Raman band comprising vibrations specific to tertiary protein structure with tryptophan residues, an IR band specific to the presence of β-protein structure and a specific IR band indicative of the presence of compounds generated during oxidative stress.

In another particular embodiment, the diagnostic method of the invention comprises obtaining a Raman band comprising vibrations specific to β-protein structure, a Raman band of amyloid peptides comprising vibrations specific to angular deformation of the peptide bond, a Raman band comprising vibrations specific to α-helix protein structure, a Raman band comprising vibrations specific to tertiary protein structure with tryptophan residues, an IR band specific to the presence of β-protein structure and a specific IR band indicative of the presence of compounds generated during oxidative stress.

In a particular embodiment of the diagnostic methods of the invention, the plasma sample is platelet-free plasma. In another particular embodiment of the diagnostic methods of the invention, the plasma sample is platelet-rich plasma. In a particular embodiment of the diagnostic methods of the invention, the plasma sample is dehydrated plasma. In another particular embodiment, the reference sample is plasma obtained from a control patient, in an additional particular embodiment, the control patient is a patient classified with stage 1 according to the GDS scale.
Methods for identifying patients susceptible to being treated with therapy suitable for Alzheimer's disease and treatment methods for treating patients selected by means of said methods

In another aspect, the invention relates to a method for identifying a subject susceptible to receiving therapy suitable for treating Alzheimer's disease (first method for identifying subjects according to the invention) comprising determining the protein structure associated with global cognitive deterioration in Alzheimer's disease in a blood sample by means of vibrational spectroscopy method comprising the steps of:
a) recording a Raman spectrum of a previously obtained human blood sample;
b) obtaining a Raman spectral value of at least one of the following Raman spectrum regions:
   R.1. region comprised between 1600 and 1700 cm⁻¹;
   R.2. region comprised between 910 and 980 cm⁻¹;
   R.3. region comprised between 730 and 760 cm⁻¹;
   R.4. region comprised between 390 and 450 cm⁻¹;
      where said spectral value is selected from a value of an interband Raman intensity ratio, a value of the area of said spectral region and/or a frequency value.
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
   by comparison of the Raman spectral value obtained in step b with a reference spectral value which allows distinguishing between class I or II, or by multivariate analysis comparison of the Raman spectral value obtained in step b
   with class I and class II reference Raman spectral values,
wherein if the blood sample containing the protein structure is associated with cognitive deterioration in Alzheimer's disease, it is indicative that said subject is susceptible to receiving therapy suitable for treating Alzheimer's disease.

In another aspect, the invention relates to a method for identifying a subject susceptible to receiving therapy for treating Alzheimer's disease (second method for identifying subjects according to the invention), comprising determining the protein structure associated with global cognitive deterioration in Alzheimer's disease in a blood sample by means of infrared spectroscopy comprising the steps of
a) recording an infrared spectrum of a previously obtained human blood sample;
b) obtaining an infrared spectral value of at least one of the following infrared spectrum regions:
   IR.1. region comprised between 1600 and 1700 cm⁻¹;
   IR.2. region comprised between 1000 and 1150 cm⁻¹;
   where said infrared spectral value is selected from a value of the area of said spectral region and/or a percentage value of the interband areas of said spectral region;
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
   by comparison of the infrared spectral value obtained in step e with a reference infrared value which allows distinguishing between class I or II, or by multivariate analysis comparison of the infrared value obtained in step e with class I or II reference infrared spectral values
wherein if the blood sample containing the protein structure is associated with cognitive deterioration in Alzheimer's disease is indicative that said subject is susceptible to receiving therapy for treating Alzheimer's disease.

The different embodiments of steps a), b) and c) according to the first and second method of the invention correspond with the embodiments described in the context of the method for determining the cognitive deterioration in Alzheimer's disease described above.

In a particular embodiment, the first method for identifying a subject susceptible to receiving therapy for treating Alzheimer's disease comprises the steps of the second method for identifying a subject susceptible to receiving therapy for treating Alzheimer's disease. In another particular embodiment of the method for identifying a subject susceptible to receiving therapy for treating Alzheimer's disease, when said method comprises the steps of the first and second method, the sample is classified by multivariate analysis comparison of the spectral values obtained in step b with class I and class II reference Raman values and with class I and class II reference infrared spectral values.

In another aspect, the invention relates to therapy suitable for treating Alzheimer's for use in treating Alzheimer's disease in a subject wherein said subject has been selected according to the first or second method for identifying a subject susceptible to receiving therapy for treating Alzheimer's disease.

In another aspect, the invention relates to a method for treating Alzheimer's disease comprising administering therapy suitable for treating Alzheimer's to a subject, wherein said subject has been selected according to the first or second method for identifying a subject susceptible to receiving therapy for treating Alzheimer's disease.

According to the present invention, "therapy suitable for treating Alzheimer's" comprises any drug or treatment that allows treating Alzheimer's, i.e., a treatment for reducing, improving or eliminating symptoms of AD. In a particular embodiment, the drugs for treating AD are selected from a cholinesterase inhibitor and an N-methyl-D-aspartate (NMDA) receptor antagonist and anti β-amyloid immunotherapy.

As it is used in the present invention, "cholinesterase inhibitor" refers to a chemical compound inhibiting the cholinesterase or anticholinesterase, preventing the destruction of the released acetylcholine, thereby causing an increase in the concentration and in the duration of the effects of the neurotransmitter. The full cholinesterase sequence in humans has accession number P06276 in the Uniprot database (2 August 2012).

There is evidence from pre-clinical studies and some studies in human beings that cholinesterase inhibition affects basic processes that have been involved in the pathogenesis of AD. For example, the acetylcholinesterase (AChE) inhibition can affect the expression of AChE isoforms and increase the expression of nicotine receptors, both being correlated with cognitive improvements in patients with AD. AChE inhibition has also been shown to affect amyloid precursor protein (APP) processing and attenuate the toxicity induced by Aβ by means of mechanisms including the interruption of Aβ production, the alteration of Aβ 1-40 and Aβ 1-42 levels and the formation of the soluble form of the amyloid precursor protein. Therefore, by way of non-limiting illustration, therapies suitable for treating mild to moderate AD, comprise cholinesterase inhibitors such as Cognex® (tacrine), Aricept (donepezil), Exelon® (rivastigmine) or Razadine® (galantamine), among others. The moderate to severe AD can be treated with Namenda® (memantine), which acts as NMDA antagonist receptors. Since NMDA antagonists work differently from those of cholinesterase inhibitors, both types of medicaments can be prescribed in combination. These medicaments can help in delaying or preventing symptoms of AD from worsening. The therapies according to the present invention additionally include music therapy, physical therapy, psychomotor education, occupational therapy or therapy with animals, among others.

Three cholinesterase inhibitors for treating Alzheimer's disease, are currently sold worldwide, specifically donepezil, galantamine and rivastigmine. Donepezil (1-benzyl-4-[(5,6-dimethoxy-1-indanon)-2-yl]-methyl-piperidine) is a reversible acetylcholinesterase (AChE) inhibitor. Galantamine([4aS-(4aα,6β,8aR*)]-4a,5,9,10,11,12-hexahydro-3-methoxy-11-methyl-6H-benzofuro[3a,3,2-ef] benzace pin-6-ol) is an alkoid isolated from snowdrop, *Galanthus nivalis.* It is a highly selective, reversible and competitive acetylcholinesterase inhibitor. Rivastigmine ((S)-N-ethyl-3-[1-(dimethylamino)ethyl]-N-methyl-phenyl-carbamate) is a reversible, non-competitive acetylcholinesterase and butyrylcholinesterase inhibitor.

Clinical and/or pre-clinical trials for other cholinesterase inhibitors, specifically for tacrine hydrochloride (Cl-970, THA.HCl), huperzine A, acotiamide, dimebolin, DEBIO 9902, IN 101, phenserine tartrate, R-phenserine, stacofylline hydrochloride (S-9977, S-9977-2), NP-61, bisnorcymserine, COL-204, SPH 1371, SPH 1373, SPH 1375, SP 04, CM 2433, metrifonate, 7-methoxytacrine (7 MEOTA), P 11149, Arisugacin, FR 152558, HUP 13, isovanihuperzine A (IVHA), MHP 133, NP 7557, P 10358, P 11012, physostigmine salicylate, velnacrine maleate (HP-029, P83-6029A), epastigmine tartrate (L-693487), ipidacrine, zanapezil, ganstigmine, icopezil maleate (CP-118954, CP-118954-11), KW 5092, quilostigmine (HP-290, NXX-066), SM 10888, T 82, TAK 802, zifrosilone MDL-73745), BGC 201259, CHF 2060, Cl 1002, E 2030, ER 127528, ET 142, F 3796, huprine X, MF 247, MF 268 bitartrate, MF 8615, P 26, PD 142012, RO 465934, SS 20, thiatolserine, tolserine tartrate, UR 1827. (ADIS R&D Insight, the 25/02/2010), have also been conducted and the foregoing could be used for treating AD according to the invention.

Additional compounds for inhibiting cholinesterases have been described, for example edrophonium, demecarium, ambenonium, neostigmine bromide, dehydroevodiamine chloride, eseroline, imperatorin, scopoletin (SCT), huperizine A (Hup A), heptylstigmine tartrate (MF-201), suronacrine maleate (HP-128), UCB-1 1056, berberine iodide, norpyridostigmine, quilostigmine (HP-290, NXX-066), THB-013, PD-142676, terestigmine tartrate (CHF-2060), thiacymserine, MF-8615, MF-268 bitartrate, anseculin hydrochloride (KA-672.HCl), ensaculin hydrochloride, icopezil maleate (CP-118954), eserine salicylate, physostigmine salicylate, JWS-USC-75IX, P11467, P-10358, bis(7)-tacrine, HMR-2420, CP-126998, TV-3279, MSF, THA-C8, subergorgic acid, suberogorgin, SPH-1286, huperzine B (Hup B), pyridostigmine bromide (Ro-1-5130), huprine Y, coronaridine, RS-1233, kobophenol A, bis(12)-huperine, RS-1259, ITH-4012, TK-19, T-81, TH-171, TH-185, distigmine bromide (BC-51), (-)-9-dehydrogalanthaminium bromide, memoquin, scopoletin 7-O-beta-D-glucopyranoside (NSC-404560), scopolyn (SCN), scopoloside, BW-284c51, withaferin A (NSC-101088), withaferine (NSC-273757), (+)-corynoline, corynoline, (S)-(-)-oxypeucedanin, oxypeucedanin, (-)-voacangine, carbomethoxyibogaine, voacangine, dieckol, phlorofucofuroeckol (PFF), phlorofucofuroeckol A, (-)-3-0-acetylspectaline hydrochloride and rhaphiasaponin 1, or salts, free bases, racemates or enantiomers thereof, and they could be used for treating AD according to the invention.

As it is used in the present invention, "NMDA receptor antagonist" refers to a chemical compound inhibiting the reaction generated by the polysynaptic discharge of nociceptive primary afferent fibers. Memantine (3,5-dimethyltricyclo[3.3.1.13,7]decan-1-amine or 3,5-dimethyladamantane-1-amine) stands out among antagonists.

Other NMDA antagonists are nimodipine (3-(2-methoxyethyl)5-propan-2-yl 2,6-dimethyl-4-(3-nitrophenyl)-1,4-dihydropyridine-3,5-dicarboxylate), dizocilpine, AP-5 (2-amino-5-phosphonopentanoic acid), AP-7 (2-amino-7-phosphonoheptanoic acid), CHF 3381 (n-(2-indanyl)-glycinamide hydrochloride]) and ifenprodil (4-[2-(4-benzylpiperidin-1-yl)-1-hydroxypropyl]phenol) and they could be used for treating AD according to the invention.

As it is used in the present invention, "anti β-amyloid immunotherapy", refers to a therapy using the immune system for slowing or stopping the damages caused by β-amyloid accumulation. Said immunotherapy comprises the administration of anti beta-amyloid antibodies. Anti beta-amyloid antibodies useful for treatment according to the present invention include those which directly destroy the amyloid plaque (microglia are stimulated by immunization and devour the already previously formed amyloid plaques), those which capture the amyloid (formation of the antigen-antibody complex in peripheral blood sequesters the amyloid out of the brain and prevents deposition) or those inhibiting aggregation (formation of the antigen-antibody complex prevents the amyloid from aggregating in senile plaques). Alternatively, anti β-amyloid immunotherapy comprises the use of immunogenic compositions comprising one or several β-amyloid peptides (for example Aβ(1-5), Aβ(1-6), Aβ(1-12), Aβ(13-28), Aβ(25-35), Aβ(35-42), Aβ(33-42) and Aβ(33-40)) (the expression Aβ(X-Y) refers to a peptide derived from the beta-amyloid peptide consisting of the amino acids at position X to the amino acid at position Y). The beta-amyloid peptides can be administered either in the form of conjugates (for example, conjugated to KLH or an albumin) or in the form of a composition with an adjuvant (for example, Freund's complete adjuvant, Freund's incomplete adjuvant, QS21, aluminum hydroxide gel, MF59, calcium phosphate, liposyn, saponin, squalene, L121, monophosphoryl lipid A (MPL) in Emulsigen, polysorbate 80, cholera toxin (CT), LTK and LTK63). By way of illustrative example, a vaccine useful in the present invention would be the AN-1972 vaccine, which consists of ßA₄₂ being an artificial copy of the human beta-amyloid protein, which when injected stimulates the immune system to clean abnormal deposits from the brain of sick rodents. This stimulates the formation of antibodies which adhere to the neuritic plaques to later be digested by the cellular elements of the immune system (microglia, astrocytes).

Alternatively, it is possible to use gamma secretase inhibitors, gamma secretase being an enzyme involved in β amyloid synthesis. Gamma secretase inhibitors useful in the present invention are, among others, LY-450139 (CAS no. 425386-60-3), also referred to as Semagacestat, DAPT (CAS no. 208255-80-5), CTS-21166 or MK 8931.

In another aspect, the invention relates to a method for identifying a subject susceptible to receiving therapy suitable (third method for identifying subjects according to the invention) for treating Alzheimer's disease comprising
a) measuring a Raman spectrum of a plasma sample from said subject obtaining at least one Raman band selected from the group consisting of a Raman band comprising vibrations specific to β-protein structure, a Raman band of amyloid peptides comprising vibrations specific to angular deformation of the peptide bond, a Raman band comprising vibrations specific to α-helix protein structure and a Raman band comprising vibrations specific to tertiary protein structure with tryptophan residues and
b) comparing the Raman spectrum obtained in step a) with the spectrum of a reference sample
wherein a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum and/or a Raman spectrum variation indicative of an increase in the vibrations specific to tryptophan residues in a tertiary protein structure with respect to the reference spectrum is indicative of the subject being susceptible to receiving therapy suitable for treating Alzheimer's disease.

In another aspect, the invention relates to a method for identifying a subject susceptible to receiving therapy suitable for treating Alzheimer's (fourth method for identifying subjects according to the invention) comprising the steps of:
a) measuring an IR spectrum of a plasma sample from said subject obtaining at least one band specific to the presence of β-protein structure and/or in at least one specific band indicative of the presence of compounds generated during oxidative stress and
b) comparing the IR spectrum obtained in step (a) with the spectrum of a reference sample
wherein an IR spectrum variation indicative of an increase in β-protein structure with respect to the reference spectrum and/or an IR spectrum variation indicative of an increase in the concentration of compounds generated in the sample during oxidative stress with respect to the reference spectrum is indicative of the patient being susceptible to receiving therapy suitable for treating Alzheimer's having Alzheimer's disease.

In another aspect, the invention relates to a method for identifying a subject susceptible to receiving therapy suitable (fifth method for identifying a subject) for treating Alzheimer's disease comprising
a) measuring a Raman spectrum of a plasma sample from said subject obtaining at least one Raman band selected from the group consisting of a Raman band comprising vibrations specific to β-protein structure, a Raman band of amyloid peptides comprising vibrations specific to angular deformation of the peptide bond, a Raman band comprising vibrations specific to α-helix protein structure and a Raman band comprising vibrations specific to tertiary protein structure with tryptophan residues,
b) measuring an IR spectrum of a plasma sample from said subject obtaining at least one band specific to the presence of β-protein structure and/or in at least one specific band indicative of the presence of compounds generated during oxidative stress and
c) comparing the Raman spectrum obtained in step a) with the spectrum of a reference sample and the IR spectrum obtained in step b) with the spectrum of a reference sample
wherein a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum, a Raman spectrum variation indicative of an increase in the vibrations specific to tryptophan residues in a tertiary protein structure with respect to the reference spectrum, an IR spectrum variation indicative of an increase in β-protein structure with respect to the reference spectrum and/or an IR spectrum variation indicative of an increase in the concentration of compounds generated in the sample during oxidative stress with respect to the reference spectrum is indicative of the patient being susceptible to receiving therapy suitable for treating Alzheimer's having Alzheimer's disease.

The different embodiments of steps a), b) and c) according to the third, fourth and/or fifth method of the invention correspond with the embodiments described in the context of the diagnostic methods for diagnosing Alzheimer's disease or for determining the cognitive deterioration in Alzheimer's disease described in the preceding section.

In another aspect, the invention relates to therapy suitable for treating Alzheimer's in a subject wherein said subject has been selected according to the third or fourth method for identifying a subject.

In another aspect, the invention relates to a method for treating Alzheimer's comprising administering therapy suitable for treating Alzheimer's to a subject, wherein said subject has been selected according to the third or fourth method for identifying a subject.

The particularities of the therapy suitable for treating Alzheimer's have been mentioned above and are also applicable.

In the present specification, the method for determining protein structure associated with global cognitive deterioration in Alzheimer's disease in a blood sample by means of Raman spectroscopy, by means of infrared spectroscopy or by means of Raman spectroscopy combined with infrared spectroscopy, the method for designing customized therapy for an individual and the diagnostic method according to any of the values defined above, as well as any of the preferred embodiments, can be referred to in the present specification as methods of the present invention.

### Apparatus of the invention

In another aspect, the invention relates to an apparatus for plasma sample analysis comprising:
(i) a receptacle for receiving a plasma sample,
(ii) at least one spectrometer selected from a Raman spectrometer and an IR spectrometer
(iii) a computer system comprising means for implementing a diagnostic method of the invention.

The devices forming the Raman spectrometer are known in the state of the art, basically a laser and a CCD (charge-coupled devices) photomultiplier. The equipment can be formed by two interchangeable monochromatic light sources (for example He-Ne laser and Ar laser) connected to an excitation optical fiber guiding the light to the optical head and the latter focusing the light on the samples. The light scattered by the sample is collected through the same optical head and by means of the collection optical fiber is guided to the monochromator which spatially and spectrally separates it. The CCD detects the signal diffracted by the monochromator and transforms the scattered light photons into a digital electric signal and the spectrum is forwarded to a computer. The purpose of the computer system of the apparatus of the invention is, among others, to control the acquisition of the scattered Raman signal and its subsequent analysis, including the graphic and mathematical processing. The computer system also performs control of the parameters of the CCD, the monochromator and the alignment of the laser, among others. The computer system can additionally allow a certain degree of manipulation of the spectra, such as for example correction of the baseline, comparison between several spectra, calculations of areas and Fourier transforms, comparison of the spectral value of the sample to be analyzed with the reference spectral value or multivariate analysis comparison with values that allow differentiating between a blood sample containing the protein structure associated with non-cognitive deterioration in Alzheimer's disease and a blood sample containing the protein structure associated with cognitive deterioration in Alzheimer's disease, among others.

In a particular embodiment, the apparatus of the invention comprises a Raman spectrometer and an IR spectrometer. The IR spectrometer can be a dispersive or Fourier transform IR spectrometer. In a preferred embodiment, when the apparatus of the invention comprises an IR spectrometer, said IR spectrometer is a Fourier transform spectrometer.

A Fourier transform spectrometer (FTIR) has three basic elements: a light source, a Michelson interferometer and a detector. The Fourier transform instruments do not contain scattering elements and detect and measure all wavelengths simultaneously. Instead of a monochromator, it uses an interferometer to produce the interference patterns containing the infrared spectra information. FTIR spectrometers use the same type of sources as dispersive instruments. The transducers are, commonly, triglycine sulfate, which is a pyroelectric transducer, or mercury cadmium telluride, which is a photoconductive transducer. To obtain the radiant energy as a function of the wavelength, the interferometer modulates the signal from the source such that it can be decoded by means of the mathematical Fourier transform technique. This operation requires a high-speed computer to perform the calculations.

In another particular embodiment, the apparatus of the invention additionally comprises a microscope.

### Computer systems, computer programs and data carriers

In another aspect, the invention relates to a computer program comprising a code suitable for performing the diagnostic methods of the invention.

In another aspect, the invention relates to a data carrier containing the computer program of the invention.

In another aspect, the invention relates to a computer system provided with means for implementing the diagnostic methods or the methods for identifying patients according to the invention. The computer system can include:
(a) at least one memory containing at least one computer program suitable for controlling the operation of the computer system for implementing a method including: (i) receiving Raman and/or IR spectra data about the sample from the patient and (ii) assigning the patient as a healthy subject or as an Alzheimer's disease patient based on the degree of identity between the spectrum obtained from the sample from the subject and the spectrum of the reference subject and
(b) at least one processor for running the computer program. Another aspect of the present invention relates to a computer program for controlling a computer system for performing the steps according to the first, second or third method of the invention.

The computer system can include one or more general processors or processors having particular purposes and associated memory, including volatile and non-volatile memory devices.

The machine-readable physical storage media useful in several embodiments of the invention can include any machine-readable physical storage medium, for example solid state memory (such as flash memory), machine-readable magnetic and optical storage media and devices, and memory using other persistent storage technologies. In some embodiments, a machine-readable medium can be any tangible medium which allows the computer to access computer programs and data. The machine-readable media can include erasable or non-erasable tangible volatile and non-volatile media implemented in any method or technology that can store information, such as machine-readable instructions, program modules, programs, data, data structures, and database information. In some embodiments of the invention, machine-readable medium includes but is not limited to RAM (Random Access Memory), ROM (Read-only Memory), EPROM (Erasable Programmable Read-only Memory), EEPROM (Electrically Erasable Programmable Read-only Memory), flash memory or other memory technology, CD-ROM (Compact Disc Read-only Memory), DVD (Digital Versatile Discs) or other optical storage medium, magnetic cassettes, magnetic tape, magnetic disc storage or other magnetic storage medium, other types of volatile and non-volatile memory, and any other tangible medium that can be used to store information and can be machine-read, including any suitable combination of the foregoing.

The present invention can be implemented in an autonomous computer or as part of a network computer system.

In some embodiments of the present invention, the Raman and/or IR spectra used as references can be used to record, register and retrieve electronically or digitally.

In some embodiments of this aspect and all the other aspects of the present invention, the system can compare the data in a "comparison module" which can use a variety of software formats and programs available for operative comparison for comparing spectra determined in the determination module with reference data. In another embodiment, the comparison module is configured for using pattern recognition techniques for comparing information of sequences of one or more inputs with one more reference data patterns. The comparison module can be configured to use existing commercially available or disposable software for comparing patterns and can be optimized for particular data comparisons that are performed.

In some embodiments, the comparison module provides a machine-readable comparison result that can be processed in the machine-readable form by means of predefined criteria, or user-defined criteria, to provide a report comprising content based in part on the comparison result that can be stored and accessed as required by a user using a display module. In some embodiments, a display module allows displaying content based in part on the comparison result for the user, wherein the content is a report indicative of the results of the comparison of the spectrum obtained from the sample from the patient of interest with the spectrum of a healthy subject.

In some embodiments, the display module allows displaying a report or content based in part on the comparison result for the end user, wherein the content is a report indicative of the results of the comparison of the spectrum of the patient with the reference spectrum. In some embodiments of this aspect and all other aspects of the invention, the comparison module, or any other module of the invention, can include an operating system (for example, UNIX, Windows) in which a relational database management system, a World Wide Web application and a World Wide Web server are run. The World Wide Web application can include the executable code necessary for generating database language instructions [for example, standard query language (SQL) instructions].

The computer instructions can be implemented in software, firmware or hardware and include any type of programmed step undertaken by modules of the information processing system. The computer system can be connected to a Local Area Network (LAN) or a Wide Area Network (WAN).

In some embodiments of this aspect and all other aspects of the present invention, a comparison module provides machine-readable data that can be processed in machine-readable manner by means of predefined criteria, or user-defined criteria, to provide retrieved content that can be stored and accessed as required by a user using a display module.

According to some embodiments of the invention, the computerized system can include or be operatively connected to a display module, such as a computer monitor, a touch screen or video display system. The display module allows presenting the user instructions to the system user, allowing the user to see system inputs and allowing the system to show the results to the user as part of a user interface. The computerized system can optionally include or be operatively connected to a printing device to make printed copies of the information outputted by the system.

In some embodiments of the present invention, a World Wide Web browser can be used to provide a user interface for allowing the user to interact with the system to enter information, to make requests and to show the retrieved content. Furthermore, the various functional modules of the system can be adapted for using a web browser to provide a user interface. By using a web browser, a user can make requests to retrieve data from data sources, such as databases, and interact with the comparison module to make comparisons and pattern matching. The user can indicate and click on user interface elements such as buttons, drop-down menus, displacement lines, etc., conventionally used in graphical user interfaces for interacting with the system and making the system perform the methods of the invention. The requests made with the user web browser can be transmitted in a network to a Web application which can process or format the request to conduct a query in one or more databases that can be used to provide the relevant information relating to the spectra generated, the content retrieved, process this information and generate the results.

### Additional inventive aspects of the present invention

The present invention refers to the following inventive aspects:

In a first aspect, the invention relates to a vibrational spectroscopy method for determining protein structure associated with global cognitive deterioration in Alzheimer's disease in a blood sample comprising the following steps:
a) recording a Raman spectrum of a previously obtained human blood sample;
b) obtaining a Raman spectral value of at least one of the following Raman spectrum regions:
   R.1. region comprised between 1600 and 1700 cm⁻¹;
   R.2. region comprised between 910 and 980 cm⁻¹;
   R.3. region comprised between 730 and 760 cm⁻¹;
   R.4. region comprised between 390 and 450 cm⁻¹;
      where said spectral value is selected from a value of an interband Raman intensity ratio, a value of the area of said spectral region and/or a frequency value.
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
   by comparison of the Raman spectral value obtained in step b
with a reference spectral value which allows distinguishing between class I or II, or by multivariate analysis comparison of the Raman spectral value obtained in step b with class I and class II reference Raman spectral values.

In a second aspect, the invention relates to a vibrational spectroscopy method for determining protein structure associated with global cognitive deterioration in Alzheimer's disease in a blood sample comprising the following steps:
a) recording an infrared spectrum of a previously obtained human blood sample;
b) obtaining an infrared spectral value of at least one of the following infrared spectrum regions:
   IR.1. region comprised between 1600 and 1700 cm⁻¹;
   IR.2. region comprised between 1000 and 1150 cm⁻¹;
      where said infrared spectral value is selected from a value of the area of said spectral region and/or a percentage value of the interband areas of said spectral region;
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
      by comparison of the infrared spectral value obtained in step b with a reference infrared value which allows distinguishing between class I or II, or by multivariate analysis comparison of the infrared value obtained in step b with class I or II reference infrared spectral values.

In a particular embodiment of the method according to inventive aspect 1, it additionally comprises the steps of a method according to inventive aspect 2 applied to a sample from the same subject. In a more particular embodiment, the sample is classified by multivariate analysis comparison of the spectral values obtained in step b with class I and class II reference Raman values and with class I and class II reference infrared spectral values.

In another third aspect, the invention relates to a method for identifying a subject susceptible to receiving therapy suitable for treating Alzheimer's disease comprising determining the protein structure associated with global cognitive deterioration in Alzheimer's disease in a blood sample by means of vibrational spectroscopy method comprising the steps of
a) recording a Raman spectrum of a previously obtained human blood sample;
b) obtaining a Raman spectral value of at least one of the following Raman spectrum regions:
   R.1. region comprised between 1600 and 1700 cm⁻¹;
   R.2. region comprised between 910 and 980 cm⁻¹;
   R.3. region comprised between 730 and 760 cm⁻¹;
   R.4. region comprised between 390 and 450 cm⁻¹;
      where said spectral value is selected from a value of an interband Raman intensity ratio, a value of the area of said spectral region and/or a frequency value.
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
      by comparison of the Raman spectral value obtained in step b with a reference spectral value which allows distinguishing between class I or II, or by multivariate analysis comparison of the Raman spectral value obtained in step b
      with class I and class II reference Raman spectral values
   wherein if the blood sample containing the protein structure is associated with cognitive deterioration in Alzheimer's disease, it is indicative that said subject is susceptible to receiving therapy suitable for treating Alzheimer's disease

In a fourth aspect, the invention relates to a method for identifying a subject susceptible to receiving therapy for treating Alzheimer's disease comprising determining the protein structure associated with global cognitive deterioration in Alzheimer's disease in a blood sample by means of infrared spectroscopy comprising the steps of
a) recording an infrared spectrum of a previously obtained human blood sample;
b) obtaining an infrared spectral value of at least one of the following infrared spectrum regions:
   IR.1. region comprised between 1600 and 1700 cm⁻¹;
   IR.2. region comprised between 1000 and 1150 cm⁻¹;
      where said infrared spectral value is selected from a value of the area of said spectral region and/or a percentage value of the interband areas of said spectral region;
c) classifying the blood sample in one of the following classes:
   I. blood sample containing the protein structure associated with non-cognitive deterioration in Alzheimer's disease;
   II. blood sample containing the protein structure associated with cognitive deterioration in Alzheimer's disease;
      by comparison of the infrared spectral value obtained in step e with a reference infrared value which allows distinguishing between class I or II, or by multivariate analysis comparison of the infrared value obtained in step e
      with class I or II reference infrared spectral values
   wherein if the blood sample containing the protein structure is associated with cognitive deterioration in Alzheimer's disease, it is indicative that said subject is susceptible to receiving therapy for treating Alzheimer's disease.

In a particular embodiment, the method according to the third inventive aspect additionally comprises the steps of a method according to the fourth inventive aspect applied to a sample from the same subject.

A fifth inventive aspect relates to therapy suitable for treating Alzheimer's for use in treating Alzheimer's disease in a subject wherein said subject has been selected according to a method according to any of the third or fourth inventive aspects.

In a particular embodiment of the methods, the blood sample is a blood plasma fraction sample.

In a particular embodiment of the methods of the first, third or fifth inventive aspects, the spectral value of the Raman spectrum region defined in R.1 comprises the ratio of Raman intensities obtained around 1671 cm⁻¹ and 1658 cm⁻¹, the spectral value of the Raman spectrum region defined in R.4 comprises the ratio of Raman intensities obtained around 409 cm⁻¹ and 423 cm⁻¹, the spectral value of the Raman spectrum region defined in R.2 comprises the area of the Raman spectral profile comprised between 910 and 980 cm⁻¹, and/or the spectral value of the Raman spectrum region defined in R.3 is selected from the group consisting of the ratio of Raman intensities obtained at the maximum of the band around 758 cm⁻¹ and at the maximum of the band located in the 740-750 cm⁻¹ region or of the frequency of the band comprised in said Raman spectrum region between 740 and 750 cm⁻¹.

In a particular embodiment of the methods of the second, fourth and fifth inventive aspects, the spectral value of the infrared spectrum region defined in IR.1 comprises the percentage of the area of the spectral profile between 1640 and 1623 cm⁻¹ with respect to the area of the amide I region between 1670-1623 cm⁻¹ expressed in second derivatives and/or the spectral value of the infrared spectrum region defined in IR.2 comprises the percentage of the area of the spectral profile between 1150 and 1000 cm⁻¹ with respect to the area in the 3010 - 2800 cm⁻¹ region.

In a particular embodiment of the methods of the first, third or fifth inventive aspects, step b comprises obtaining the spectral values of the Raman spectrum regions defined in R.1, R.2, R.3 and R.4; and where the classification of step c comprises multivariate analysis comparison of said spectral values with a class I reference Raman spectral value and with a class II reference Raman spectral value, wherein said reference spectral values are comparable to those obtained in step b.

In a particular embodiment of the methods, step b comprises obtaining the spectral values of the Raman spectrum regions defined in R.1, R.2, R.3 and R.4; obtaining the spectral values of the infrared spectrum regions defined in IR.1 and IR.2; and step c comprises classifying by means of multivariate analysis comparison of said spectral values with a class I reference Raman spectral value, with a class II reference Raman spectral value, class I reference infrared spectral value and with a class II reference infrared spectral value, wherein said reference spectral values are comparable to those obtained in step b.

In another more particular aspect, the class II reference value comprises at least one reference spectral value of a blood sample of the following sub-classes:
II-a. blood sample associated with mild cognitive deterioration in Alzheimer's disease, with a value of GDS-3 on Reisberg's global cognitive deterioration scale.
II-b. blood sample associated with mild cognitive deterioration in Alzheimer's disease, with a value of GDS-4 on Reisberg's global cognitive deterioration scale.
II-c. blood sample associated with mild cognitive deterioration in Alzheimer's disease, with a value of GDS-5 on Reisberg's global cognitive deterioration scale.
II-d. blood sample associated with mild cognitive deterioration in Alzheimer's disease, with a value of GDS-6 on Reisberg's global cognitive deterioration scale.
II-e. blood sample associated with mild cognitive deterioration in Alzheimer's disease, with a value of GDS-7 on Reisberg's global cognitive deterioration scale.

In another particular aspect, the reference spectral value is obtained under the same conditions described in steps a-b according to inventive aspects 1 or 2, from reference blood samples as they are defined in class I and in class II, or from reference blood samples as they are defined in class I, in sub-class II-a, in sub-class II-b, in sub-class II-c, in sub-class II-d and in sub-class II-e.

In another particular aspect, the multivariate analysis is a discriminant analysis using the following as independent variables:
- the spectral value or values obtained in step b, and the reference spectral values;
   and the following as a grouping variable:
- a variable indicative of classes I and II of the reference spectral values.

In another particular aspect, the blood plasma fraction sample is prepared for recording the Raman spectrum following a process comprising:
a) completely evaporating to dryness a volume of a blood plasma fraction to obtain at least between 1 and 3 mg of a dry solid residue of said fraction, where the evaporation of said fraction volume is performed at a temperature comprised between 2 and 25°C, preferably between 15°C and 25°C.
b) collecting the previous dry fraction and transferring it, by means of a conventional micromortar for FT-Raman spectroscopy, to a cylindrical aluminum pan with a semi-spherical hollow of 2 mm in diameter.

In another particular aspect of the methods of the invention, when it further comprises recording the infrared spectrum of a blood plasma fraction sample, said plasma sample is prepared for recording its infrared spectrum following a process comprising extending a volume of between 3 and 7 µL of a blood plasma fraction on a ZnSe crystal and leaving it to completely evaporate to dryness at a temperature comprised between 2 and 25°C, preferably between 15°C and 25°C.

In another particular aspect, the blood plasma fraction is obtained from a previously obtained human peripheral blood sample by means of a blood fractionation process by centrifugation-filtration.

In another particular aspect, the methods of the invention are for obtaining an indication of the presence or absence of a condition of Alzheimer's disease from the analysis of a human blood sample.

In a sixth aspect, the invention relates to a diagnostic method for diagnosing Alzheimer's disease in a subject comprising the steps of:
a) measuring a Raman spectrum of a plasma sample from said subject obtaining at least one Raman band selected from the group consisting of a Raman band comprising vibrations specific to β-protein structure, a Raman band of amyloid peptides comprising vibrations specific to angular deformation of the peptide bond, a Raman band comprising vibrations specific to α-helix protein structure and a Raman band comprising vibrations specific to tertiary protein structure with tryptophan residues and
b) comparing the Raman spectrum obtained in step a) with the spectrum of a reference sample
wherein a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum and/or a Raman spectrum variation generated by vibrations specific to tryptophan residues in a tertiary protein structure with respect to the reference spectrum is indicative of the patient having Alzheimer's disease.

In a seventh aspect, the invention relates to a method for identifying a subject susceptible to receiving therapy suitable for treating Alzheimer's disease comprising
a) measuring a Raman spectrum of a plasma sample from said subject obtaining at least one Raman band selected from the group consisting of a Raman band comprising vibrations specific to β-protein structure, a Raman band of amyloid peptides comprising vibrations specific to angular deformation of the peptide bond, a Raman band comprising vibrations specific to α-helix protein structure and a Raman band comprising vibrations specific to tertiary protein structure with tryptophan residues and
b) comparing the Raman spectrum obtained in step a) with the spectrum of a reference sample
wherein a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum and/or a Raman spectrum variation generated by vibrations specific to tryptophan residues in a tertiary protein structure with respect to the reference spectrum is indicative of the subject being susceptible to receiving therapy suitable for treating Alzheimer's disease.

In a particular embodiment of the sixth and seventh aspects, the Raman band comprising vibrations specific to beta sheet protein is the band around 1671 cm⁻¹, the Raman band comprising vibrations specific to angular deformation of the peptide bond is the band around 409 cm⁻¹, the Raman band comprising vibrations specific to α-helix protein structure is defined by the spectral profile between 980 and 910 cm⁻¹ and/or the Raman band comprising vibrations specific to tryptophan residues in a tertiary protein structure is the band around the 740-750 cm⁻¹ region.

In another particular aspect
- the Raman spectrum variation indicative of an increase in intensities specific to β-protein structure with respect to the reference spectrum is an increase in the ratio between the intensities located around 1671 cm⁻¹ and 1658 cm⁻¹,
- the Raman spectrum variation indicative of an increase in intensities specific to tryptophan residues in a tertiary protein structure with respect to the reference spectrum is an increase in the ratio between the intensity of the maximum of the band located around 758 cm⁻¹ and the intensity of the band located around 743 cm⁻¹ or an increase in the frequency of the band in the range 740-750 cm⁻¹,
- the Raman spectrum variation indicative of a reduction in intensities specific to α-helix protein structure with respect to the reference spectrum is a reduction in the area of the spectral profile between 980-910 cm⁻¹
   and/or
- the Raman spectrum variation indicative of an increase in vibrations specific to angular deformation of the peptide bond with respect to the reference spectrum is an increase in the ratio between the intensities of the bands located around 409 cm⁻¹ and 423 cm⁻¹.

In another particular aspect of the methods of the invention, the Raman spectrum is recorded using a near-infrared laser excitation line.

In an eighth aspect, the invention relates to a diagnostic method for diagnosing Alzheimer's disease in a subject comprising the steps of:
a) measuring an IR spectrum of a plasma sample from said subject obtaining at least one band specific to the presence of β-protein structure and/or in at least one specific band indicative of the presence of compounds generated during oxidative stress and
b) comparing the IR spectrum obtained in step (a) with the spectrum of a reference sample
wherein an IR spectrum variation indicative of an increase in β-protein structure with respect to the reference spectrum and/or an IR spectrum variation indicative of an increase in the concentration of compounds generated in the sample during oxidative stress with respect to the reference spectrum is indicative of the patient having Alzheimer's disease.

In a ninth aspect, the invention relates to a method for identifying a subject susceptible to receiving therapy suitable for treating Alzheimer's comprising the steps of:
a) measuring an IR spectrum of a plasma sample from said subject obtaining at least one band specific to the presence of β-protein structure and/or in at least one specific band indicative of the presence of compounds generated during oxidative stress and
b) comparing the IR spectrum obtained in step (a) with the spectrum of a reference sample
wherein an IR spectrum variation indicative of an increase in β-protein structure with respect to the reference spectrum and/or an IR spectrum variation indicative of an increase in the concentration of compounds generated in the sample during oxidative stress with respect to the reference spectrum is indicative of the patient being susceptible to receiving therapy suitable for treating Alzheimer's having Alzheimer's disease.

In a tenth aspect, the invention relates to therapy suitable for treating Alzheimer's for use in a method for treating Alzheimer's disease in a subject wherein said subject has been selected according to a method according to any of the eighth or ninth inventive aspects.

In a particular embodiment, the band specific to the presence of β-protein structure is the band around 1640-1623 cm⁻¹ and/or the band specific to the presence of compounds generated during oxidative stress is the band between 1150 cm⁻¹ and 1000 cm⁻¹.

In another particular embodiment, the IR spectrum variation indicative of an increase in β-protein structure with respect to the reference spectrum is an increase in the spectral profile area in the region between 1640-1623 cm⁻¹ and/or the IR spectrum variation indicative of an increase in the concentration of compounds generated in the sample during oxidative stress with respect to the reference spectrum is an increase in the spectral profile area in the region between 1150 cm⁻¹ and 1000 cm⁻¹.

In another more particular embodiment said increase in the spectral profile area in the region between 1640-1623 cm⁻¹ is determined as a percentage of the area in the region between 1640-1620 cm⁻¹ with respect to the area of the amide I region between 1670-1623 cm⁻¹ expressed in second derivatives and/or wherein said increase in the concentration of compounds generated during oxidative stress is determined as a percentage of the area in the region between 1150 cm⁻¹ and 1000 cm⁻¹ of the spectrum with respect to the area in the region between 3010 cm⁻¹ and 2800 cm⁻¹.

In another particular embodiment, the method according to the sixth or seventh inventive aspect additionally comprises the steps of a method according to any of the eighth or ninth inventive aspects applied to a sample from the same subject.

In another particular embodiment of the method according to any one of the inventive aspects, it comprises measuring the Raman spectrum obtaining a Raman band comprising vibrations specific to β-protein structure, a Raman band comprising vibrations specific to angular deformation of the peptide bond, a Raman band comprising vibrations specific to α-helix protein structure and a Raman band comprising vibrations specific to tertiary protein structure with tryptophan residues.
and
wherein the diagnostic method of the invention comprises measuring an IR spectrum obtaining a band specific to the presence of β-protein structure and a specific band indicative of the presence of compounds generated during oxidative stress
wherein a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum, a Raman spectrum variation indicative of a reduction in intensities specific to tryptophan residues in a protein structure with respect to the reference spectrum, an IR spectrum variation indicative of an increase in beta sheet protein structure with respect to the reference spectrum and an IR spectrum variation indicative of an increase in the concentration of compounds generated in the sample during oxidative stress with respect to the reference spectrum is indicative of the patient having Alzheimer's disease.

In a particular embodiment, the plasma sample is dehydrated plasma.

In another particular embodiment, the reference sample is plasma obtained from a control patient, preferably a patient classified with stage 1 according to the GDS scale.

In an eleventh aspect, the invention relates to an apparatus for plasma sample analysis comprising:
(i) a receptacle for receiving a plasma sample,
(ii) a Raman spectrometer and/or an IR spectrometer, and
(iii) a computer system comprising means for implementing of a diagnostic method according to the invention.

In a twelfth aspect, the invention relates to a computer program comprising a code suitable for performing a diagnostic method according to the invention.

In a thirteenth aspect, the invention relates to a data carrier containing a computer program according to the invention.

The invention is illustrated below based on the following examples provided by way of non-limiting illustration of the scope of the invention.

### Examples

### Example 1. Process for obtaining peripheral blood plasma fraction.

The following is done to obtain the plasma fraction from a blood sample obtained:
4 mL of blood obtained from venous blood extractions from healthy controls and from patients with AD are taken in a BD Vacutainer tube with heparin-lithium (green stopper, ref. 368884) and are centrifuged at 1700 g for 10 minutes at 4°C. The supernatant corresponds to the blood plasma fraction.

### Example 2. Raman and infrared spectra measurement of the blood plasma fraction.

The Raman spectral measurements were taken following the following steps:

### a) Preparation of samples

50 µL of the plasma fraction are taken and placed in a sample holder with a cavity of this volume, and are dried at room temperature between 15 and 25°C in air flow hood. The samples can be dried at a temperature comprised between 2 and 25°C without any alteration of the samples being observed, but for the sake of faster sample evaporation, it is performed between 15 and 25°C. The resulting solid is collected from the sample holder, transferred to an agate mortar and homogenized for the subsequent Raman spectrum measurement. The resulting ground solid is transferred to an accessory conventional micro-mortar for FT-Raman spectroscopy containing a cylindrical aluminum pan with a semi-spherical hollow of 2 mm in diameter where the powder solid (1-3 mg) of blood plasma is compacted with the micro-pestle.

### b) Spectral recording conditions

The Raman spectra were measured in a Brüker RFS 100 Raman spectrometer using a line of 1064 nm of a 100 mw power neodymium-YAG laser as excitation radiation. The recording conditions were the following: recording range: 3600-300 cm⁻¹; accumulation of 1,000 spectra; spectral resolution of 4 cm⁻¹. All the spectra were obtained by optical back-reflection on the surface of the samples at room temperature and under identical analytical conditions, and therefore the intensities of the bands can be directly compared between spectra.

Figure 1 shows Raman spectra of plasma from healthy controls and patients with mild, moderate and severe AD. When considered globally, no significant differences are observed between them, and it is therefore necessary that they be limited to small spectral ranges to observe significant differences. Figure 2 shows that Raman intensity around 1671 increases when going from healthy controls to patients with AD as a result of β-sheet polypeptide structure formation. Unlike the Raman spectra of platelets of mice transgenic with AD (Chen et al., Laser Phys. Lett. 8, 547-552 (2011)), no significant reduction is observed in the intensity of the amide I band around 1654 cm⁻¹. The formation of Aβ-amyloid peptides in AD (Figure 3) is also clearly shown by the occurrence of a band around 409 cm⁻¹ which is characteristic of the Raman spectra of Aβ-40 and Aβ-42 peptides, which have been measured in this invention. This band has not yet been described in the literature, but due to considerations of group frequencies the value of its frequency leads to tentatively assigning it to polypeptide backbone angular deformation vibrations. The relative intensity of this band has been used for the first time in this invention as a discriminant parameter, among others, between healthy controls and patients with AD. As can be seen in Examples 4 and 5, the addition of its intensity to other spectral values (also referred to in this specification as spectroscopic parameters) described in the present document considerably improves sensitivity and specificity of classifying samples.

Figure 4 demonstrates for the first time that the area of the band of the spectral profile between 980 and 912 cm⁻¹ is less in patients with AD in comparison with healthy controls. This spectral change can be interpreted as a reduction in the α-helix proteins structure taking into account the Raman spectra of model polypeptides and proteins with this type of polypeptide structure.

Finally, Figure 5 shows the spectral region where the tertiary protein structures with tryptophan residues can be seen. Unlike what is observed in the Raman spectra of platelets of mice transgenic with AD (Chen et al. Laser Phys. Lett. 8, 547-552 (2011)), the band located around 740-743 cm⁻¹ does not experience an increase but rather a reduction in intensity, and it further shifts towards higher frequencies in comparison with the spectra of blood plasma from healthy controls.

The spectral infrared measurements were taken following the following steps:

### a) Preparation of samples

A volume comprised between 3 and 7 µL of the plasma fraction is taken, placed on a ZnSe crystal for infrared spectroscopy, extended with a spatula and dried at room temperature between 15 and 25°C in an air flow hood. The resulting film is measured after by infrared spectroscopy.

### b) Spectral recording conditions

The infrared spectra were measured in a Perkin-Elmer model 1725X Fourier transform spectrometer. The recording conditions were the following: recording range: 4000-400 cm⁻¹; accumulation of 32 spectra; spectral resolution of 2 cm⁻¹. The possible contribution of environmental water vapor bands was corrected by counteracting the water vapor spectrum with a suitable factor until achieving the absence of water vapor bands in the region between 2000-1800 cm⁻¹.

Figure 6A shows the mean spectra of senile control plasma and of patients with moderate AD. In the latter plasma, slightly higher absorption can be observed in the 1640-1620 cm⁻¹ range due to β-sheet protein structure. This can be more clearly seen in the spectra expressed in second derivatives. Finally, an increase in infrared absorption in the 1000-1150 cm⁻¹ region attributable to the formation of oxygenated compounds resulting from oxidative stress can be seen in patients with AD (Figure 6B).

### Examples 3-5. Discriminant analysis of Raman spectra of peripheral blood plasma

Discriminant analyses have been conducted using the group of samples indicated in Table 1. Height ratios 1671/1658, 758/743, 409/423 cm⁻¹, frequency of the band in the 740-750 cm⁻¹ range, and area of the spectral profile between 980-910 cm⁻¹ normalized with respect to the area of the amide I band were considered for this purpose. The spectra subjected to this statistical treatment were the result of correcting the baseline as shown in Figure 1. Class I or II relating to cognitive state was considered as a dependent or grouping variable (Group. var.), and the aforementioned spectroscopic parameters (spectral values) were considered as independent variables. The same probabilities were considered beforehand for both classes of samples. A cross validation was performed to predict the classification of the samples considering all the samples except one for the analytical model, and the excluded sample was used to classify it according to this model. This operation was performed for each of the samples of Table 1.

**Table 1.**

| Samples from healthy controls and patients with AD | |
|---|---|
| PATIENTS AND CONTROLS | |
| (GDS) | Total |
| Senile controls (GDS 1) | 12 |
| Mild AD (GDS 3) | 8 |
| Mild AD (GDS 4) | 9 |
| Moderate AD (GDS 5) | 7 |
| Advanced (GDS 6) | 6 |
| Advanced (GDS 7) | 5 |
| | **47** |

These discriminant analyses were performed using the corresponding SPSS computer program, version 19 (SPSS Inc., Chicago, IL, USA).

### Example 3

The samples of Table 1 were classified by means of discriminant analysis considering only the spectroscopic parameter of height ratio 1671/1658 cm⁻¹. The results included in Table 2 indicate that correct classification of the samples of around 70% is obtained by means of cross validation.

**Table 2**

| Results of the classification^{b,c} | | | | | |
|---|---|---|---|---|---|
| Group. Var. | | | Group of predicted pertinence | | Total |
| | | | 1.000000 | 2.000000 | |
| Original | Count | 1.000000 | 9 | 3 | 12 |
| | | 2.000000 | 11 | 24 | 35 |
| | % | 1.000000 | 75.0 | 25.0 | 100.0 |
| | | 2.000000 | 31.4 | 68.6 | 100.0 |
| Cross validation^{a} | Count | 1.000000 | 9 | 3 | 12 |
| | | 2.000000 | 11 | 24 | 35 |
| | % | 1.000000 | 75.0 | 25.0 | 100.0 |
| | | 2.000000 | 31.4 | 68.6 | 100.0 |

| | | | | | |
|---|---|---|---|---|---|
| a. The cross validation is applied only to the cases of the analysis. In the cross validation, each case is classified by means of the derivative functions from the remaining cases. b. 70.2% of the originally grouped cases are correctly classified. c. 70.2% of the grouped cases validated by means of cross validation are correctly classified. | | | | | |

### Example 4

The samples of Table 1 were classified by means of discriminant analysis considering the spectroscopic parameters (spectral values) of height ratio 1671/1658 cm⁻¹ and 758/743 cm⁻¹, frequency of the band in the 740-750 cm⁻¹ range and area of the spectral profile between 980 and 910 cm⁻¹. The results included in Table 3 indicate that a correct classification of the samples close to 80% is obtained by means of cross validation. Therefore, a classifying improvement is obtained in comparison with Example 3, where only the parameter of height ratio 1671/1658 cm⁻¹ is considered.

**Table 3**

| Results of the classification^{b,c} | | | | | |
|---|---|---|---|---|---|
| Group. Var. | | | Group of predicted pertinence | | Total |
| | | | 1.000000 | 2.000000 | |
| Original | Count | 1.000000 | 10 | 2 | 12 |
| | | 2.000000 | 6 | 29 | 35 |
| | % | 1.000000 | 83.3 | 16.7 | 100.0 |
| | | 2.000000 | 17.1 | 82.9 | 100.0 |
| Cross validation ^{a} | Count | 1.000000 | 9 | 3 | 12 |
| | | 2.000000 | 7 | 28 | 35 |
| | % | 1.000000 | 75.0 | 25.0 | 100.0 |
| | | 2.000000 | 20.0 | 80.0 | 100.0 |

| | | | | | |
|---|---|---|---|---|---|
| a. The cross validation is applied only to the cases of the analysis. In the cross validation, each case is classified by means of the derivative functions from the remaining cases. b. 83.0% of the originally grouped cases are correctly classified. c. 78.7% of the grouped cases validated by means of cross validation are correctly classified. | | | | | |

### Example 5

The effect of adding the spectroscopic parameter or spectral value of height ratio 409/423 cm⁻¹ on the correct classification of samples by means of cross validation is analyzed in this example in comparison with Example 4. From this point of view, the results of Table 4 clearly show that a very significant improvement (correct classification exceeding 90%) has been obtained by introducing this spectroscopic parameter relating to the presence of amyloid peptides in the samples of AD.

**Table 4.**

| Results of the classification^{b,c} | | | | | |
|---|---|---|---|---|---|
| Group. Var. | | | Group of predicted pertinence | | Total |
| | | | Healthy controls | Cases of AD | |
| Original | Count | Healthy controls | 11 | 1 | 12 |
| | | Cases AD | 2 | 33 | 35 |
| | % | Healthy controls | 91.7 | 8.3 | 100.0 |
| | | Cases AD | 5.7 | 94.3 | 100.0 |
| Cross validation^{a} | Count | Healthy controls | 11 | 1 | 12 |
| | | Cases AD | 3 | 32 | 35 |
| | % | Healthy controls | 91.7 | 8.3 | 100.0 |
| | | Cases AD | 8.6 | 91.4 | 100.0 |

| | | | | | |
|---|---|---|---|---|---|
| a. The cross validation is applied only to the cases of the analysis. In the cross validation, each case is classified by means of the derivative functions from the remaining cases. b. 93.6% of the originally grouped cases are correctly classified. c. 91.5% of the grouped cases validated by means of cross validation are correctly classified. | | | | | |

By comparing Example 5 with Example 4 it can be clearly seen that introducing this parameter very significantly improves the percentage of the correct classification of samples, going from approximately 80% to more than 90%.

### Example 6

This example analyzes the combination of infrared spectroscopy with Raman spectroscopy in the classification of samples, which is included in the following Table 5. This example has been carried out considering the five Raman spectroscopic values of Example 5 and the two spectroscopic values of infrared regions IR.1 and IR.2. The two values in the infrared refer to the percentage of the area of β-protein sheet structure measured in the amide I infrared region (region IR.1) and to the area of the infrared spectral profile between 1000 and 1150 cm⁻¹ (region IR.2).

**Table 5.**

| PERCENTAGES OF CORRECT CLASSIFICATIONS | | | |
|---|---|---|---|
| Sample size | IR | RAMAN | IR + RAMAN |
| Healthy controls (12) + mild AD (8) | 82% | 85% | 95% |
| Healthy controls (12)+ AD (35) | 62% | 92% | 92% |

Two sample size situations have been considered, namely, on one hand classification of samples by comparing healthy controls with patients with mild AD (GDS 3), and on the other hand classification of samples by comparing healthy controls with patients having any stage of AD (GDS 3-7). Table 5 shows that the combination of both spectroscopic techniques leads to a considerable improvement of the correct classification of the samples when they are from patients with mild AD, which is particularly interesting from the point of view of being able to reliably diagnose this disease in its early stages. When patients with AD with any GDS are considered globally, the combination of both techniques has no positive effect whatsoever because as also occurs with the lymphocyte fraction (Carmona et al. Anal. Bioanal. Chem. 402, 2015-2021 (2012)), the β-protein structure content as a differentiating spectroscopic parameter is greater in early stages of the disease and subsequently decreases.

## Claims

1. A vibrational spectroscopy method for determining protein structure associated with global cognitive deterioration in Alzheimer's disease in a blood sample comprising the following steps:
a) recording a Raman spectrum of a previously obtained human blood sample;
b) obtaining a Raman spectral value of at least one of the following Raman spectrum regions:
R.1. region comprised between 1600 and 1700 cm⁻¹;
R.2. region comprised between 910 and 980 cm⁻¹;
R.3. region comprised between 730 and 760 cm⁻¹;
R.4. region comprised between 390 and 450 cm⁻¹;
where said spectral value is selected from a value of an interband Raman intensity ratio, a value of the area of said spectral region and/or a frequency value.
c) classifying the blood sample in one of the following classes:
I. blood sample containing the protein structure associated with non-cognitive deterioration in Alzheimer's disease;
II. blood sample containing the protein structure associated with cognitive deterioration in Alzheimer's disease;
by comparison of the Raman spectral value obtained in step b with a reference spectral value which allows distinguishing between class I or II, or by multivariate analysis comparison of the Raman spectral value obtained in step b with class I and class II reference Raman spectral values.

2. A vibrational spectroscopy method for determining protein structure associated with global cognitive deterioration in Alzheimer's disease in a blood sample comprising the following steps:
a) recording an infrared spectrum of a previously obtained human blood sample;
b) obtaining an infrared spectral value of at least one of the following infrared spectrum regions:
IR.1. region comprised between 1600 and 1700 cm⁻¹;
IR.2. region comprised between 1000 and 1150 cm⁻¹;
where said infrared spectral value is selected from a value of the area of said spectral region and/or a percentage value of the interband areas of said spectral region;
c) classifying the blood sample in one of the following classes:
I. blood sample containing the protein structure associated with non-cognitive deterioration in Alzheimer's disease;
II. blood sample containing the protein structure associated with cognitive deterioration in Alzheimer's disease;
by comparison of the infrared spectral value obtained in step b with a reference infrared value which allows distinguishing between class I or II, or by multivariate analysis comparison of the infrared value obtained in step b with class I or II reference infrared spectral values.

3. The method according to claim 1, additionally comprising the steps of a method according to claim 2 applied to a sample from the same subject.

4. The method according to claim 3, wherein the sample is classified by multivariate analysis comparison of the spectral values obtained in step b with class I and class II reference Raman values and with class I and class II reference infrared spectral values.

5. The method according to any one of claims 1 to 4, where the blood sample is a blood plasma fraction sample.

6. The method according to any one of claims 1 and 3-5, where the spectral value of the Raman spectrum region defined in R.1 comprises the ratio of Raman intensities obtained around 1671 cm⁻¹ and 1658 cm⁻¹, the spectral value of the Raman spectrum region defined in R.4 comprises the ratio of Raman intensities obtained around 409 cm⁻¹ and 423 cm⁻¹, the spectral value of the Raman spectrum region defined in R.2 comprises the area of the Raman spectral profile comprised between 910 and 980 cm⁻¹, and/or the spectral value of the Raman spectrum region defined in R.3 is selected from the group consisting of the ratio of Raman intensities obtained at the maximum of the band around 758 cm⁻¹ and at the maximum of the band located in the 740-750 cm⁻¹ region or of the frequency of the band comprised in said Raman spectrum region between 740 and 750 cm⁻¹.

7. The method according to any one of claims 2-5, where the spectral value of the infrared spectrum region defined in IR.1 comprises the percentage of the area of the spectral profile between 1640 and 1623 cm⁻¹ with respect to the area of the amide I region between 1670-1623 cm⁻¹ expressed in second derivatives, and/or the spectral value of the infrared spectrum region defined in IR.2 comprises the percentage of the area of the spectral profile between 1150 and 1000 cm⁻¹ with respect to the area in the 3010-2800 cm⁻¹ region.

8. The method according to any one of claims 1, 3-6, where step b comprises obtaining the spectral values of the Raman spectrum regions defined in R.1, R.2, R.3 and R.4; and where the classification of step c comprises multivariate analysis comparison of said spectral values with a class I reference Raman spectral value and with a class II reference Raman spectral value, where said reference spectral values are comparable to those obtained in step b.

9. The method according to any one of claims 3-4 or 5-8, where step b comprises obtaining the spectral values of the Raman spectrum regions defined in R.1, R.2, R.3 and R.4; obtaining the spectral values of the infrared spectrum regions defined in IR.1 and IR.2; and step c comprises classifying said spectral values by means of multivariate analysis comparison with a class I reference Raman spectral value, with a class II reference Raman spectral value, with a class I reference infrared spectral value and with a class II reference infrared spectral value, where said reference spectral values are comparable to those obtained in steps b.

10. The method according to any one of claims 8 or 9, where the class II reference value comprises at least one reference spectral value of a blood sample of the following sub-classes:
II-a. blood sample associated with mild cognitive deterioration in Alzheimer's disease, with a value of GDS-3 on Reisberg's global cognitive deterioration scale.
II-b. blood sample associated with mild cognitive deterioration in Alzheimer's disease, with a value of GDS-4 on Reisberg's global cognitive deterioration scale.
II-c. blood sample associated with mild cognitive deterioration in Alzheimer's disease, with a value of GDS-5 on Reisberg's global cognitive deterioration scale.
II-d. blood sample associated with mild cognitive deterioration in Alzheimer's disease, with a value of GDS-6 on Reisberg's global cognitive deterioration scale.
II-e. blood sample associated with mild cognitive deterioration in Alzheimer's disease, with a value of GDS-7 on Reisberg's global cognitive deterioration scale.

11. The method according to any one of claims 8 or 9, where the reference spectral value is obtained under the same conditions described in steps a-b according to claims 1 or 2, from reference blood samples as they are defined in class I and in class II, or from reference blood samples as they are defined in class I, in sub-class II-a, in sub-class II-b, in sub-class II-c, in sub-class II-d and in sub-class II-e.

12. The method according to any one of claims 8 to 11, where the multivariate analysis is a discriminant analysis using the following as independent variables:
- the spectral value or values obtained in step b, and the reference spectral values;
and the following as a grouping variable:
- a variable indicative of classes I and II of the reference spectral values.

13. A diagnostic method for diagnosing Alzheimer's disease in a subject comprising the steps of:
a) measuring a Raman spectrum of a plasma sample from said subject obtaining at least one Raman band selected from the group consisting of a Raman band comprising vibrations specific to β-protein structure, a Raman band of amyloid peptides comprising vibrations specific to angular deformation of the peptide bond, a Raman band comprising vibrations specific to α-helix protein structure and a Raman band comprising vibrations specific to tertiary protein structure with tryptophan residues and
b) comparing the Raman spectrum obtained in step a) with the spectrum of a reference sample
wherein a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum and/or a Raman spectrum variation indicative of an increase in vibrations specific to tryptophan residues in a tertiary protein structure with respect to the reference spectrum, is indicative of the patient having Alzheimer's disease.

14. The method according to claim 13, wherein the Raman band comprising vibrations specific to beta sheet protein is the band approximating 1671 cm⁻¹, the Raman band comprising vibrations specific to angular deformation of the peptide bond is the band approximating 409 cm⁻¹, the Raman band comprising vibrations specific to α-helix protein structure is defined by the spectral profile between 980 and 910 cm⁻¹ and/or the Raman band comprising vibrations specific to tryptophan residues in a tertiary protein structure is the band approximating the 740-750 cm⁻¹ region.

15. The method according to any one of claims 13 to 14, wherein
- the Raman spectrum variation indicative of an increase in intensities specific to β-protein structure with respect to the reference spectrum is an increase in the ratio between the intensities located around 1671 cm⁻¹ and 1658 cm⁻¹,
- the Raman spectrum variation indicative of an increase in intensities specific to tryptophan residues in a tertiary protein structure with respect to the reference spectrum is an increase in the ratio between the intensity of the maximum of the band located around 758 cm⁻¹ and the intensity of the band located around 743 cm⁻¹ or an increase in the frequency of the band in the 740-750 cm⁻¹ range,
- the Raman spectrum variation indicative of a reduction in intensities specific to α-helix protein structure with respect to the reference spectrum is a reduction in the area of the spectral profile between 980-910 cm⁻¹
and/or
- the Raman spectrum variation indicative of an increase in vibrations specific to angular deformation of the peptide bond with respect to the reference spectrum is an increase in the ratio between the intensities of the bands located around 409 cm⁻¹ and 423 cm⁻¹.

16. A diagnostic method for diagnosing Alzheimer's disease in a subject comprising the steps of:
a) measuring an IR spectrum of a plasma sample from said subject obtaining at least one band specific to the presence of β-protein structure and/or in at least one specific band indicative of the presence of compounds generated during oxidative stress and
b) comparing the IR spectrum obtained in step (a) with the spectrum of a reference sample
wherein an IR spectrum variation indicative of an increase in β-protein structure with respect to the reference spectrum and/or an IR spectrum variation indicative of an increase in the concentration of compounds generated in the sample during oxidative stress with respect to the reference spectrum is indicative of the patient having Alzheimer's disease.

17. The method according to claim 16, wherein the band specific to the presence of β-protein structure is the band approximating 1640-1623 cm⁻¹ and/or the band specific to the presence of compounds generated during oxidative stress is the band between 1150 cm⁻¹ and 1000 cm⁻¹.

18. The method according to any of claims 16 or 17, wherein the IR spectrum variation indicative of an increase in β-protein structure with respect to the reference spectrum is an increase in the spectral profile area in the region between 1640-1623 cm⁻¹ and/or the IR spectrum variation indicative of an increase in the concentration of compounds generated in the sample during oxidative stress with respect to the reference spectrum is an increase in the spectral profile area in the region between 1150 cm⁻¹ and 1000 cm⁻¹.

19. The method according to claim 18, wherein said increase in the spectral profile area in the region between 1640-1623 cm⁻¹ is determined as a percentage of the area in the region between 1640-1620 cm⁻¹ with respect to the area of the amide I region between 1670-1623 cm⁻¹ expressed in second derivatives and/or wherein said increase in the concentration of compounds generated during oxidative stress is determined as a percentage of the area in the region between 1150 cm⁻¹ and 1000 cm⁻¹ of the spectrum with respect to the area in the region between 3010 cm⁻¹ and 2800 cm⁻¹.

20. The method according to any one of claims 13 to 15, additionally comprising the steps of a method according to any of claims 16 to 19 applied to a sample from the same subject.

21. The method according to claim 20, wherein the method according to any one of claims 13 to 15 comprises measuring the Raman spectrum obtaining a Raman band comprising vibrations specific to β-protein structure, a Raman band comprising vibrations specific to angular deformation of the peptide bond, a Raman band comprising vibrations specific to α-helix protein structure and a Raman band comprising vibrations specific to tertiary protein structure with tryptophan residues.
and
wherein the method according to any one of claims 16 to 19 comprises measuring an IR spectrum obtaining a band specific to the presence of β-protein structure and a specific band indicative of the presence of compounds generated during oxidative stress
wherein a Raman spectrum variation indicative of an increase in intensities specific to β-protein structure with respect to the reference spectrum, a Raman spectrum variation indicative of an increase in intensities specific to angular deformation of the peptide bond with respect to the reference spectrum, a Raman spectrum variation indicative of a reduction in intensities specific to α-helix protein structure with respect to the reference spectrum, a Raman spectrum variation indicative of a reduction in intensities specific to tryptophan residues in a protein structure with respect to the reference spectrum, an IR spectrum variation indicative of an increase in beta sheet protein structure with respect to the reference spectrum, and an IR spectrum variation indicative of an increase in the concentration of compounds generated in the sample during oxidative stress with respect to the reference spectrum is indicative of the patient having Alzheimer's disease.
